# EUROPEAN PATENT SPECIFICATION

(11) **EP 2 547 795 B1**
(45) Date of publication and mention of the grant of the patent: **14.01.2015**
(21) Application number: 11756928.5
(22) Date of filing: 16.03.2011
(51) Int. Cl.: C12Q 1/68, C12N 15/11

(54) **COMPOSITIONS AND METHODS FOR THE DETECTION OF GENOMIC FEATURES**
ZUSAMMENSETZUNGEN UND VERFAHREN ZUR ERKENNUNG VON GENOM-MERKMALEN
COMPOSITIONS ET PROCÉDÉS PERMETTANT DE RECHERCHER DES CARACTÉRISTIQUES GÉNOMIQUES

(30) Priority: 16.03.2010 US 314491 P
(43) Date of publication of application: 23.01.2013
(73) Proprietor: Nanostring Technologies, Inc, Seattle, WA 98109 (US)
(72) Inventor: HENGEN, Paul, Nevin, Pasadena, CA 91104 (US); GEISS, Gary, K., Seattle, WA 98117 (US); ELLIOT, Nathan, A., Seattle, WA 98102 (US); WEBSTER, Phillppa, Jane, Seattle, WA 98103 (US)
(74) Representative: Mintz Levin Cohn Ferris Glovsky and Popeo LLP
(86) International application number: PCT/US2011/028657
(87) International publication number: WO 2011/116088

(56) References cited:
- WO-A2-2007/076129
- WO-A2-2007/076132
- WO-A2-2008/121828
- WO-A2-2010/059731
- US-A1- 2010 047 924
- GEISS GARY K ET AL: "Direct multiplexed measurement of gene expression with color-coded probe pairs", NATURE BIOTECHNOLOGY, NATURE PUBLISHING GROUP, NEW YORK, NY, US, vol. 26, no. 3, 1 March 2008 (2008-03-01), pages 317-325, XP002505107, ISSN: 1087-0156, DOI: 10.1038/NBT1385
- NEWKIRK HEATHER L ET AL: "Determination of genomic copy number with quantitative microsphere hybridization", HUMAN MUTATION, JOHN WILEY & SONS, INC, US, vol. 27, no. 4, 1 April 2006 (2006-04-01), pages 376-386, XP002544643, ISSN: 1059-7794, DOI: 10.1002/HUMU.20312
- JEON, J. ET AL.: 'A comprehensive profile of DNA copy number variations in a Korean population: identification of copy number invariant regions among Koreans' EXPERIMENTAL AND MOLECULAR MEDICINE. vol. 41, no. 9, September 2009, pages 618 - 628, XP008164442

## Description

This invention relates generally to the field of molecular biology, and
specifically, to the fields of detection, identification, and quantification of target nucleic acid molecules in mixtures.

### BACKGROUND OF THE INVENTION

Gene copy number and chromosomal number variations can have profound effects on biological functions. These variations are at the core of altered developmental, physiologic and pathologic processes. Therefore, identifying and quantifying the copy number of genes and chromosomes in subjects, in particular, in pre-natal subjects, can aid the early detection of pathology.

Nucleic acids can be detected and quantified based on their specific polynucleotide sequences. The basic principle underlying existing methods of detection and quantification is the hybridization of a labeled complementary probe sequence to a target sequence of interest in a sample. The formation of a duplex indicates the presence of the target sequence in the sample.

This technique, called molecular hybridization, has been a useful tool for identifying and analyzing specific nucleic acid sequences in complex mixtures. This technique has been used in diagnostics, for example, to detect nucleic acid sequences of various microbes in biological samples. In addition, hybridization techniques have been used to map genetic differences or polymorphisms between individuals. Furthermore, these techniques have been used to monitor changes in gene expression in different populations of cells or in cells treated with different agents.

For example, Geiss et al ("Direct Multiplexed Measurement of Gene Expression with Color-Coded Probe Pairs", Nature Biotechnology 26(3): p317 - 325 (2008)) discusses the NanoString nCounter gene expression system. mRNA expression is profiled using two sequence specific probes for each gene of interest: the first (capture) probe contains a 35 - 50 base sequence complementary to a particular target mRNA, coupled to an affinity tag such as biotin; the second (reporter) probe contains a second 35-50 base sequence complementary to the target mRNA, coupled to a color-coded tag that provides a detection signal.

Another two-probe method is disclosed in WO2008/121828. This method for calculating copy number employs a first probe of known length (L1) specific to a control genomic sequence, and a second probe of known length (L2) specific to a target sequence, and comprises measuring the total length of the hybridization signal corresponding to the first and second probes. Calculation of copy number is dependent on L1 and L2 and the total length of the hybridization signal of first and second probes. Comparison of the signals from both the genomic control sequence and the target sequence is therefore required.

Thus, there exists a need for more accurate, quicker and more sensitive detection, identification and quantification of copy number of genes and chromosomes. Particularly, there exists a need for the specific detection of gene copy number and chromosome copy cumber in complex mixtures and multiplex reactions.

### SUMMARY OF THE INVENTION

The invention provides a method of determining the copy number of a DNA sequence in a genome comprising providing a first sample containing genomic DNA; fragmenting the genomic DNA; denaturing the genomic DNA; providing a reference sample comprising fragmented genomic DNA wherein the copy number of the fragment of genomic DNA to be detected is known; providing a first nanoreporter comprising a first probe and a second probe wherein the first probe comprises a first label attachment region to which are attached one or more label monomers that emit light constituting a first signal; a second label attachment region, which is non-over-lapping with the first label attachment region, to which are attached one or more label monomers that emit light constituting a second signal; and a first target-specific sequence attached to the first probe, wherein the target-specific sequence specifically hybridizes to the genomic DNA sequence to be detected; and wherein the second probe comprises
(i) a second target-specific sequence, wherein the second target-specific sequence specifically hybridizes to the fragmented genomic DNA sequence to be detected; and
(ii) a first affinity tag, wherein the first and second target specific sequences hybridize to different regions of the same fragmented genomic DNA sequence to be detected; contacting the first probe and the second probe with the fragmented genomic DNA of the first sample and the reference sample wherein the contact is made under conditions sufficient for hybridization of the first and second target specific sequences to a fragment of the fragmented genomic DNA comprising the genomic DNA sequence to be detected to form a first hybridized complex;
purifying the first hybridized complex through the first affinity tag, thereby separating the first hybridized complex from unbound materials; stretching the first hybridized complex using a flow-stretch, receding meniscus, or electro-stretch technique, thereby spatially separating said label monomers; measuring a signal from the first probe, wherein said signal uniquely identifies the at least one fragment of the fragmented genomic DNA comprising the genomic DNA sequence to be detected; and comparing the signal from the first sample to the signal from the reference sample, wherein the copy number of the first sample is determined by correlating the signal from the first sample with the signal from the reference sample.

In another embodiment, the genomic DNA is mammalian genomic DNA. Specifically, the mammal is a human.

In another embodiment, the genomic DNA sample is unamplified. In another embodiment, hybridization is performed in solution.

In another embodiment, the fragmentation is performed by restriction enzyme digestion. Specifically, the restriction enzyme is Alul or Bfa1. In another embodiment, the fragmentation is performed chemically, enzymatically (for example, using one or more restriction endonucleases or a DNAase), by mechanical shearing or sonication.

In another embodiment, the signal generated from the first probe hybridized to the at least one fragment of the fragmented genomic DNA comprising the genomic DNA sequence to be detected comprises about the same unit signal, or multiple thereof. In another embodiment, the signal generated from the first probe hybridized to the at least one fragment of the fragmented genomic DNA comprising the genomic DNA sequence to be detected comprises a mixture of two or more different label monomers.

In another embodiment, said labels are fluorescent.

The reference sample can be a synthetic nucleic acid sample or a biological genomic DNA sample.

The invention also provides a method further comprising normalizing the signal generated as described above, by the steps of providing at least a second nanoreporter comprising a third probe and a fourth probe, wherein the third probe comprises a third label attachment region to which are attached one or more label monomers that emit light constituting a third signal; a fourth label attachment region, which is non-over-lapping with the third label attachment region, to which are attached one or more label monomers that emit light constituting a fourth signal; and a third target-specific sequence attached to the third probe, wherein the target-specific sequence specifically hybridizes to a first DNA fragment from a copy number invariant region of the genome; and wherein the fourth probe comprises
(i) a fourth target-specific sequence, wherein the fourth target-specific sequence specifically hybridises to the first DNA fragment from a copy number invariant region of the genome; and
(ii) a second affinity tag wherein the third target specific sequence of the third probe and the fourth target-specific sequence of the fourth probe hybridize to different regions of the same fragment from a copy number invariant region of the genome;
contacting the third probe and the fourth probe with the fragmented genomic DNA from the first sample and the reference sample wherein the contact is made under conditions sufficient for hybridization of the third target specific sequence and the fourth target specific sequence to the first DNA fragment from a copy number invariant region of the genome to form a second hybridized complex; purifying the second hybridized complex through the second affinity tag, thereby separating the second hybridized complex from unbound materials; stretching the third probe hybridized to the first DNA fragment from a copy number invariant region of the genome using a flow-stretch, receding meniscus, or electro-stretch technique, thereby spatially separating said label monomers; measuring a signal from the third probe, wherein said signal uniquely identifies the first DNA fragment from a copy number invariant region of the genome; and comparing the signal from the the second nanoreporter contacted with the first sample and the second nanoreporter contacted with the reference sample, wherein the number of multiples of the quantity of signal from the second nanoreporter contacted with the first sample compared to the quantity of signal from the second nanoreporter contacted with the reference sample normalizes the signal from the first nanoreporter contacted with the first sample.

In another embodiment, the first DNA fragment from a copy number invariant region of the genome comprises a nucleic acid sequence selected from the group consisting of SEQ ID NOs: 1-66. In another embodiment, the first DNA fragment from a copy number invariant region of the genome comprises a nucleic acid sequence selected from the group consisting of SEQ ID NOs: 2, 5, 7, 12, 13, 17, 19, 24, 25, 28, 32, 36, 38, 40, 44, 46, 50, 52, 56, 58, 62 and 66. In another embodiment, the first DNA fragment from a copy number invariant region of the genome comprises a nucleic acid sequence selected from the group consisting of SEQ ID NOs: 2, 5, 13, 19, 28, 46, 50, 56, 58 and 66.

In another embodiment, the first probe further comprises an affinity tag. In another embodiment, the genomic target DNA is mammalian genomic DNA. Specifically, the mammal is a human.

In another embodiment, the signal generated from the second probe hybridized to the first control DNA sequence comprises a mixture of two or more different label monomers.

In another embodiment, the labels or label monomers are fluorescent.

The reference sample can be a synthetic nucleic acid sampler or a biological genomic DNA sample.

The invention also provides a method as described above for detecting two or more DNA sequences in a genome further comprising:
(a) providing two or more nanoreporters that each specifically hybridise to a distinct genomic DNA sequence to be detected;
(b) measuring a signal from the two or more nanoreporters, wherein said signal uniquely identifies each of the corresponding distinct genomic DNA sequences thereby detecting two or more DNA sequences in a genome; and
(c) determining the copy number by comparing the signal from the first sample to the signal from the reference sample.

Various embodiments of this method include the following. The two or more nanoreporters can be single or dual nanoreporters.

The genomic DNA is mammalian genomic DNA. Further, the mammal can be a human.

The genomic DNA sample can be unamplified. The contacting step can be performed in solution.

The fragmentation can be performed by restriction enzyme digestion. Restriction enzymes to be used include Alul and Bfal. The fragmentation can also be performed chemically, by mechanical shearing or sonication.

Various embodiments of this method include the following. The copy number invariant sequence specific nanoreporter can be a single or dual nanoreporter.

The first DNA fragment from a copy number invariant region of the genome comprises a nucleic acid sequence can be any of SEQ ID Nos: 1-66. Preferably, the first DNA fragment from a copy number invariant region of the genome comprises a nucleic acid sequence can be any of SEQ ID NOs: 2, 5, 7, 12, 13, 17,19, 24, 25,28, 32, 36, 38, 40, 44,46, 50, 52, 56, 58, 62 and 66. More preferably, the first DNA fragment from a copy number invariant region of the genome comprises a nucleic acid sequence can be any of SEQ ID NOs: 2, 5, 13, 19, 28, 46, 50, 56, 58 and 66.

The genomic DNA is mammalian genomic DNA. Further, the mammal can be a human.

The signal generated from the two or more nanoreporters hybridized to each of the fragmented genomic DNAs comprising the genomic DNA sequence to be detected can include a mixture of two or more different label monomers. The signal generated from the invariable sequence specific nanoreporter can include a mixture of two or more different label monomers.

Further, the labels or label monomers can be fluorescent.

In any of the above methods, more than one invariable sequence specific nanoreporter may be used. The invariable sequences these nanoreporters specifically hybridize to can include any of SEQ ID NOs:1-66. As few as 1 and as many as 100 invariable sequence specific nanoreporters may be used at once. These nanoreporters may all specifically bind different invariable sequences. In one assay, 1, 2, 3, 4, 5, 6, 7, 8, 9, or 10 invariable sequence specific nanoreporters may be used.

The invention also provides a kit, comprising
(a) a first nanoreporter comprising a first probe and a second probe, wherein said first probe comprises
   (i) a first label attachment region to which are attached one or more label monomers that emit light constituting a first signal;
   (ii) a second label attachment region, which is non-over-lapping with the first label attachment region, to which are attached one or more label monomers that emit light constituting a second signal; and
   (iii) a first target-specific sequence attached to the first probe, wherein the first target-specific sequence specifically hybridises to a target DNA sequence;
   and wherein said second probe comprises
   (i) a second target-specific sequence, wherein the first target-specific sequence specifically hybridises to the target DNA sequence; and
   (ii) a first affinity tag;
      wherein the first target specific sequence of the first probe and the second target specific sequence of the second probe hybridize to different regions of the same target DNA;
(b) a second nanoreporter comprising a third probe and a fourth probe, wherein said third probe comprises
   (i) a third label attachment region to which are attached one or more label monomers that emit light constituting a third signal;
   (ii) a fourth label attachment region, which is non-over-lapping with the third label attachment region, to which are attached one or more label monomers that emit light constituting a fourth signal; and
   (iii) a third target-specific sequence attached to the third probe, wherein the third target-specific sequence specifically hybridises to a first DNA fragment from a copy number invariant region of the genome;
   and wherein said fourth probe comprises
   (i) a fourth target-specific sequence, wherein the fourth target-specific sequence specifically hybridises to the first DNA fragment from a copy number invariant region of the genome; and
   (iv) a second affinity tag;
   wherein the third target specific sequence of the third probe and the fourth target-specific sequence of the fourth probe hybridize to different regions of the same fragment from a copy number invariant region of the genome; and
(c) a restriction enzyme.

In one embodiment of the kit, the first probe further comprises an affinity tag. Specifically, the restriction enzyme is Alu1 or Bfa1.

In another embodiment, said labels are fluorescent. In another embodiment, the kit further comprises a control DNA sequence of known copy number.

Further, any of the nanoreporters described above may be detected using the nCounter® system (alo referred to as the nanoreporter code system).

### BRIEF DESCRIPTION OF THE DRAWINGS

Figure 1 is a bar graph depicting detection of X chromosome copy number in comparison to Y chromosome and chromosome 18 and 21 copy number using the nCounter® system of the invention.
Figure 2 is a bar graph depicting detection of X chromosome, Y chromosome and chromosome 18 and 21 copy number using the nCounter® system of the invention.
Figure 3 is a bar graph depicting detection of X chromosome copy number using the nCounter® system of the invention.
Figure 4 is a bar graph depicting detection of kinase and GPCR DNA copy number using the nCounter® system of the invention. These data demonstrate the ability of the nCounter® system to measure fold-changes and detect 2 copies per cell with negligible error.
Figure 5 is a bar graph depicting detection of kinase and GPCR RNA molecules using the nCounter® system of the invention. These data demonstrate the ability of the nCounter® system to measure small fold-changes in other nucleic acids that are similar to DNA copy number changes expected in the CNV assay.

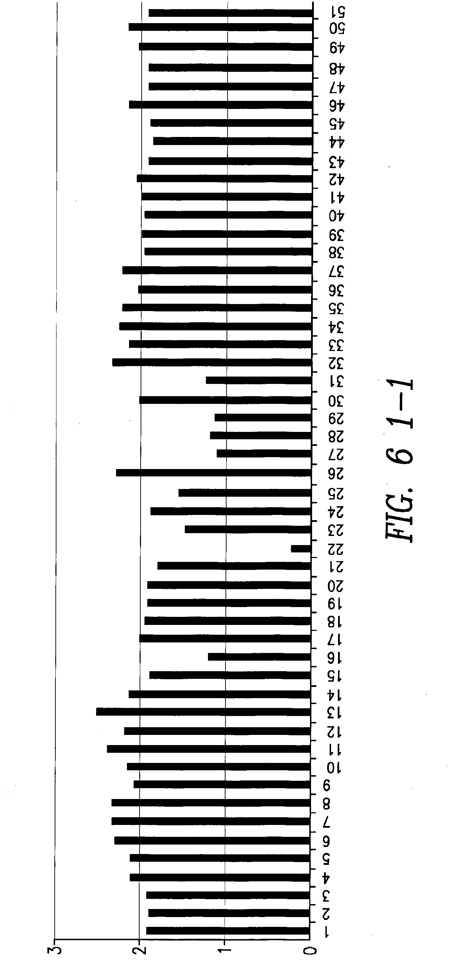

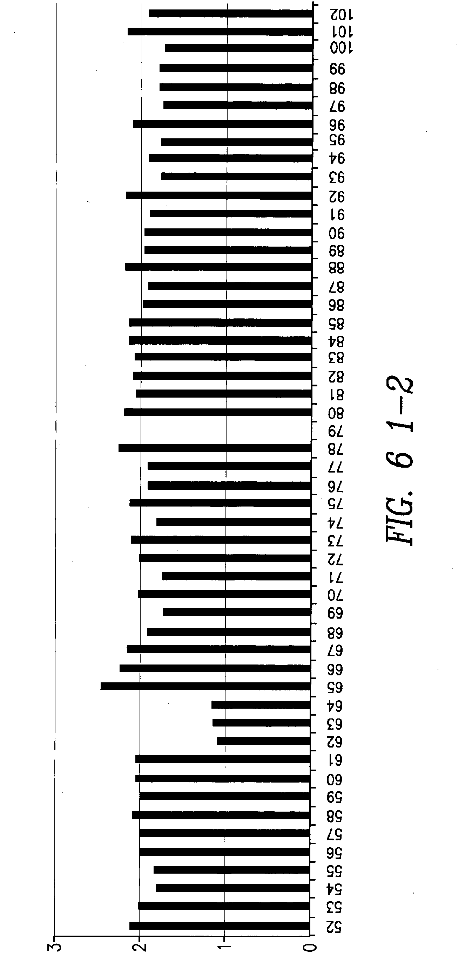

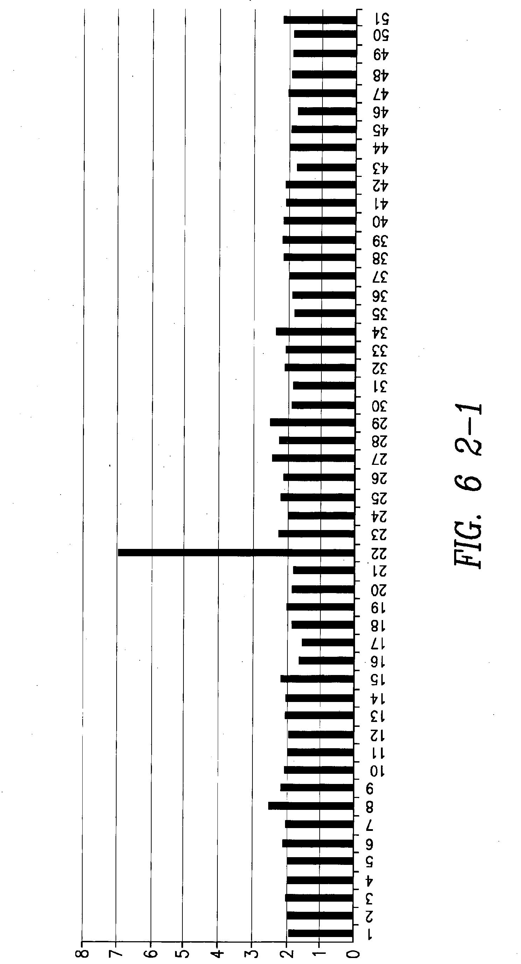

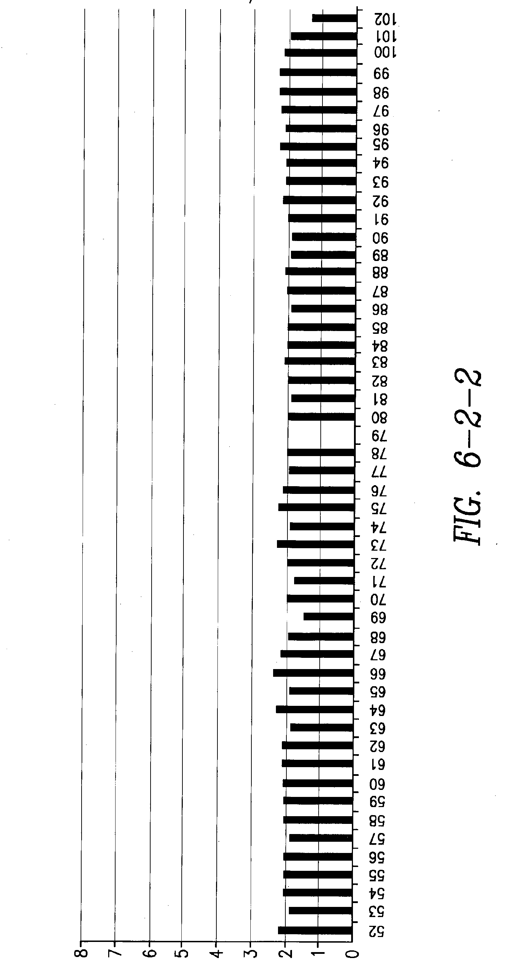

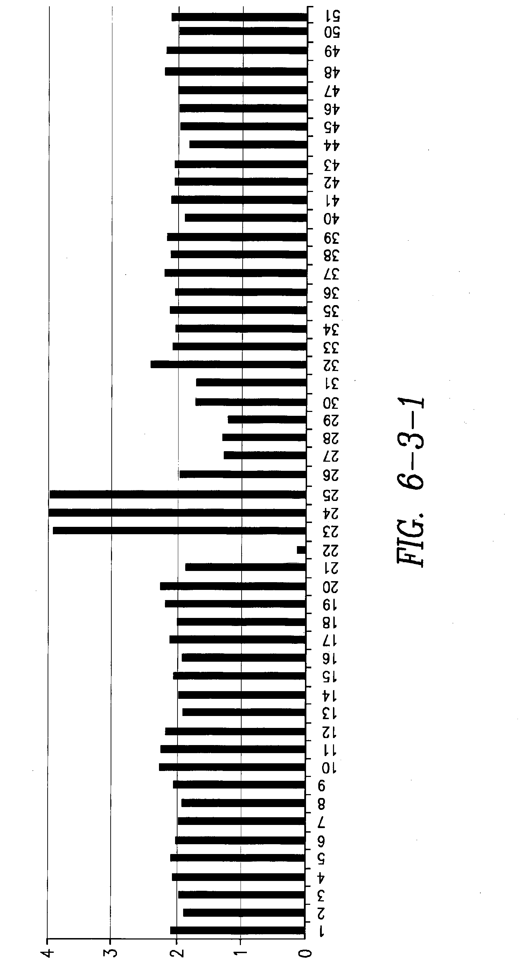

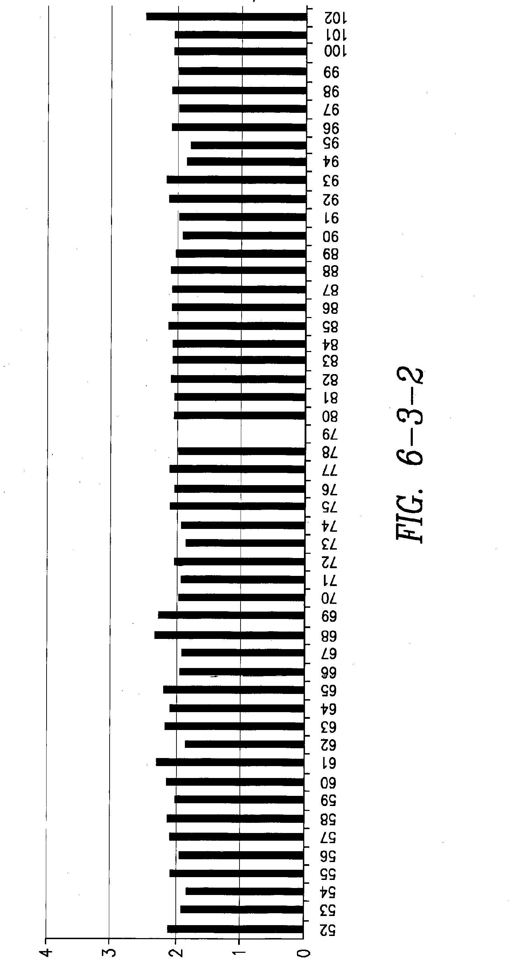

Figure 6 shows 3 bar graphs showing copy number of genomic sequences in comparison to 23 invariant genomic regions in three different samples using the nCounter® system of the invention.
Figure 7 is a line graph showing the relationship between fragment length and counts obtained via hybridization using the nCounter® system.
Figure 8 is a bar graph showing a comparison of copy number for a region of chromosome 7.
Figure 9 is a chart showing the number of copy calls for all 313 autosomal probes in the Human Karyotype Panel.

### DETAILED DESCRIPTION

The invention provides a sensitive, hybridization-based technology for determining the copy number of a given DNA sequence in a genome. For instance, the copy numbers of genes, intragenic sequences, intronic sequences, regulatory sequences (including promotor, enhancer, and repressor elements), and gene splice forms can be determined. In certain embodiments of the invention, the nCounter® Analysis System is used. In preferred embodiments, the genomic DNA to be used with the nCounter® Analysis System is fragmented.

In specific embodiments, genomic DNA is fragmented into sequences of between 100 and 5000 bp prior to use with the nCounter® Analysis System. More specifically, the fragments are on average between 100 and 1000 basepairs ("bp"), between 100 and 500 bp or between 200 and 500 bp. According to certain embodiments, the average DNA fragment size of a sample of genomic DNA for use with the nCounter® Analysis System according to the invention is within one standard deviation of 100, 200, 300, 400, 500, 600, 700, 800, 900, 1000, 1100, 1200, 1300, 1400, 1500, 1600, 1700, 1800, 1900 or 2000 bp.

In specific embodiments of the invention, fragmentation of genomic DNA is performed using enzymatic digestion, sonication, mechanical shearing or chemically. In a preferred embodiment, enzymatic digestion is performed using restriction enzymes. Preferred restriction enzymes include Alu1 which cuts as sequence AGCT. Another preferred restriction enzyme is Bfa1 which cuts at sequence CTAG. More than one restriction enzyme may be used to fragment the genomic DNA. In specific embodiments, 1, 2, 3, 4, 5, 6, 7, 8, 9, or 10 restriction enzymes are used to digest the genomic DNA.

According to certain embodiments, the genomic DNA must also be denatured from double stranded DNA to single stranded DNA. This denaturation may be done according to any method known in the art. In specific examples, the DNA is heat denatured. For example heat denaturation can be performed at 95°C for 5 minutes.

In specific embodiments of the invention, the copy number of a genomic sequence is ascertained. First, the signal from each sample is normalized by determining the relative amount of genomic DNA in each assay. This is accomplished by including a set of probes in each assay which determine the signal generated by invariant regions in the genome. "Invariant regions" are those regions of the genome whose copy number rarely differs between individuals. In a preferred embodiment, invariant regions with a copy number of two are selected. Probes to 1-9 invariant regions can be used. In another preferred embodiment, probes to ten such invariant regions are included. The amount of input DNA in each assay can be deduced from the relative signal generated by these invariant regions in each sample, and the signal from all probes in each assay can subsequently be normalized to a common amount of input DNA.

Following this sample input normalization, the copy number of any given region in an experimental sample can be determined by comparing the signal of a particular probe in the experimental sample to the signal of the same probe in a reference sample in which the copy number of the genomic target of that probe is known. The reference sample can be either a synthetic sample containing known amounts of the appropriate targets, or, in a preferred embodiment, a biological genomic DNA sample in which the copy number of the relevant regions is known.

nCounter® probes to any of the following sequences can be used as control sequences.

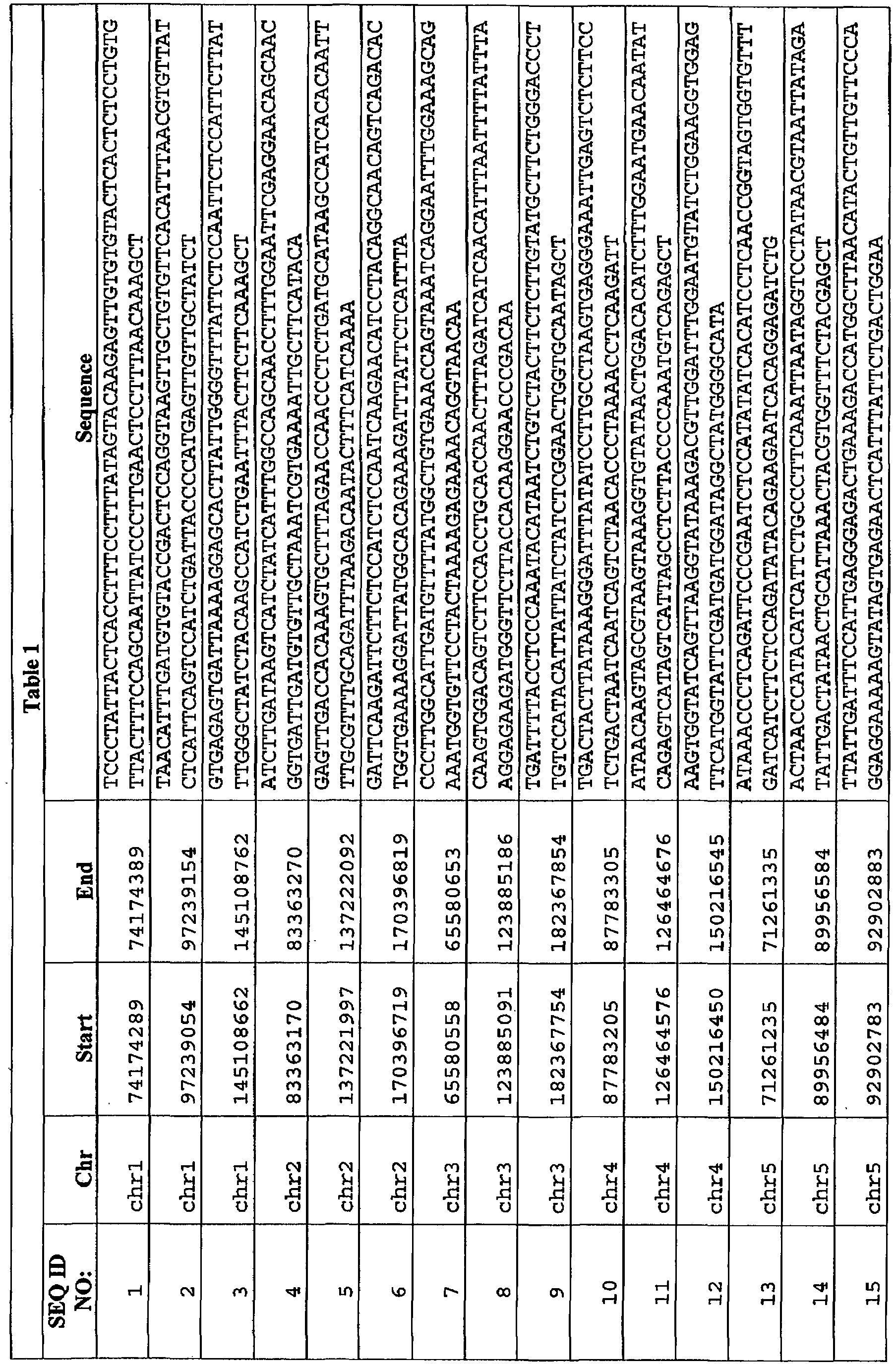

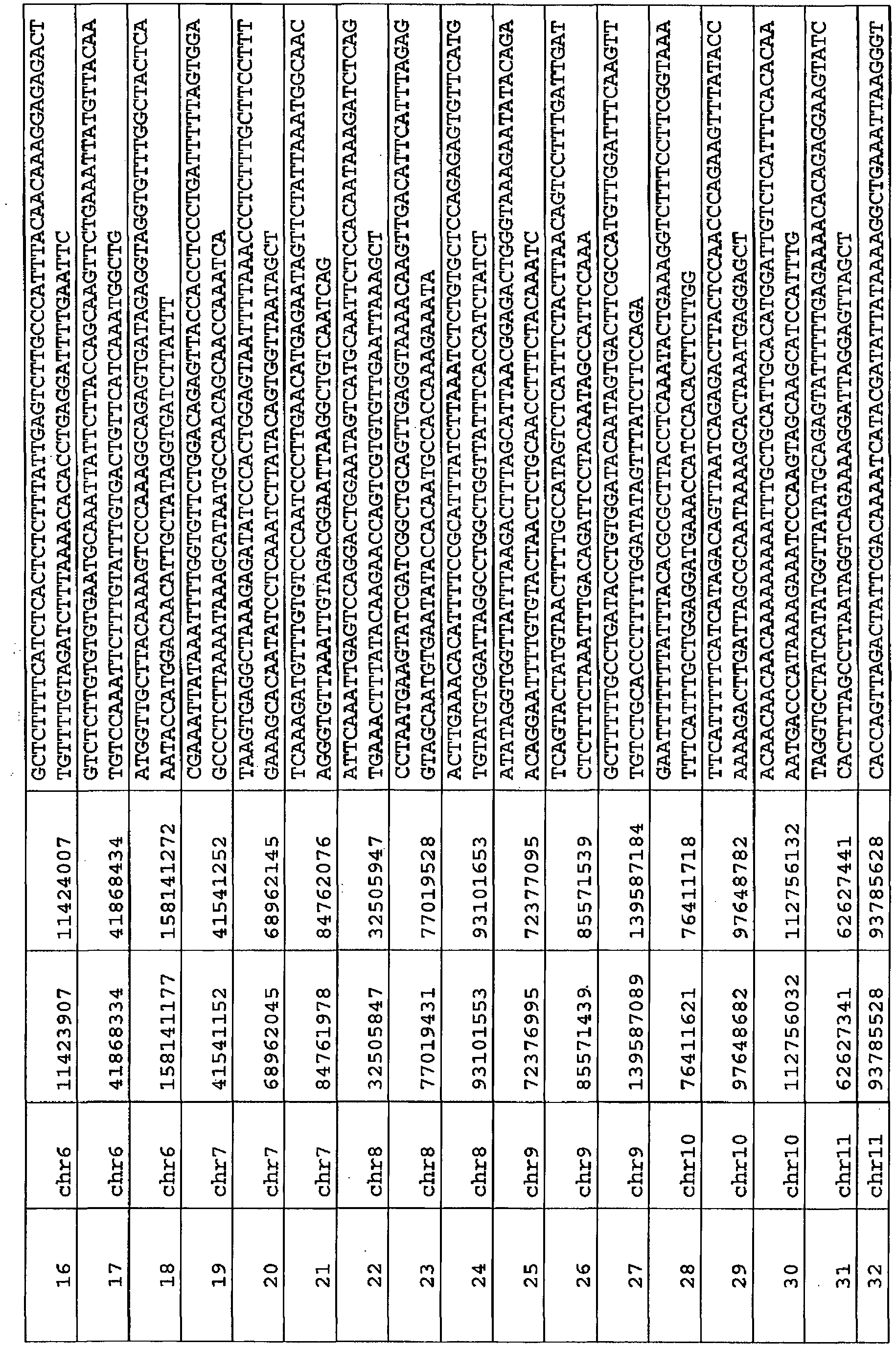

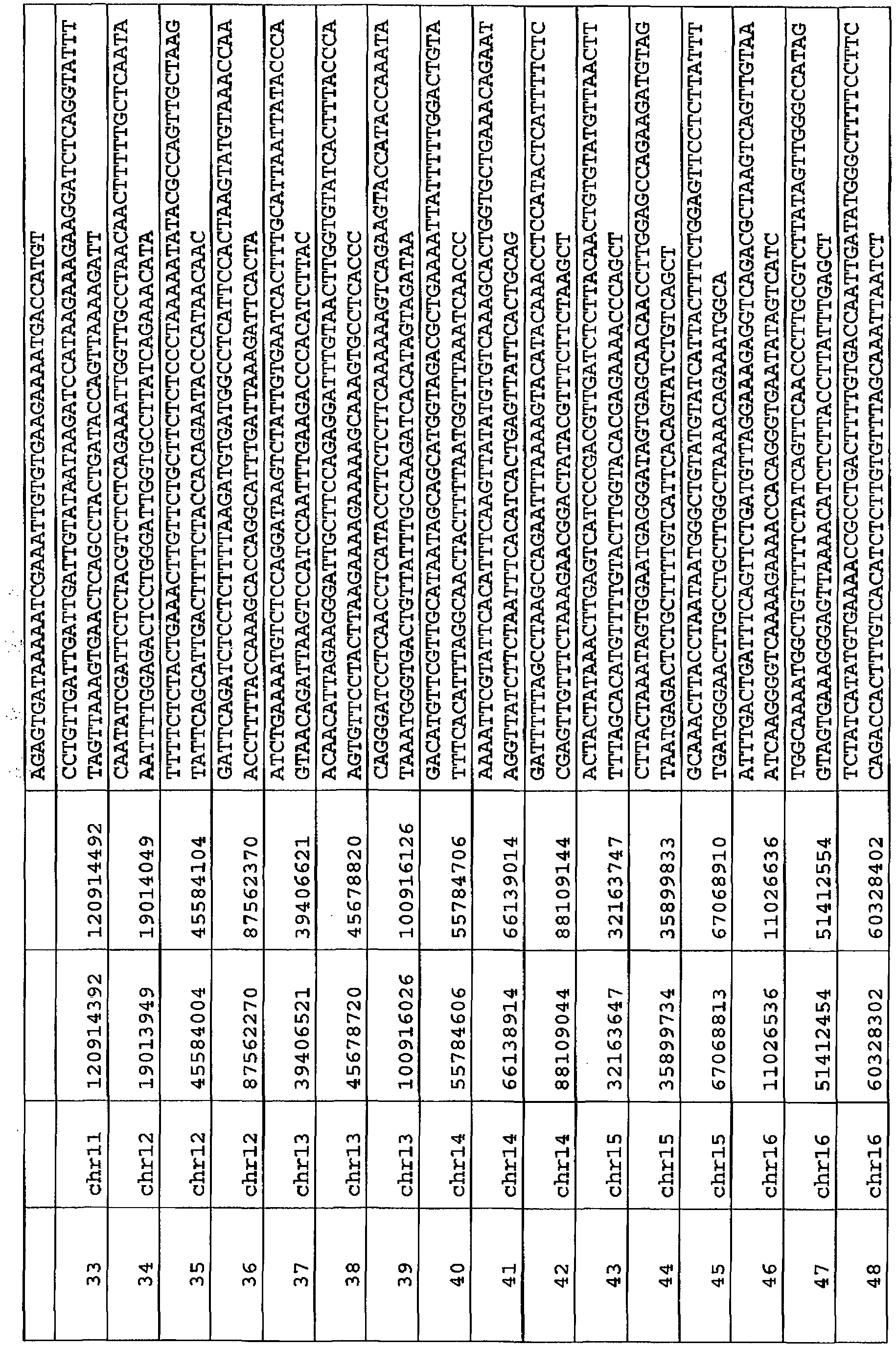

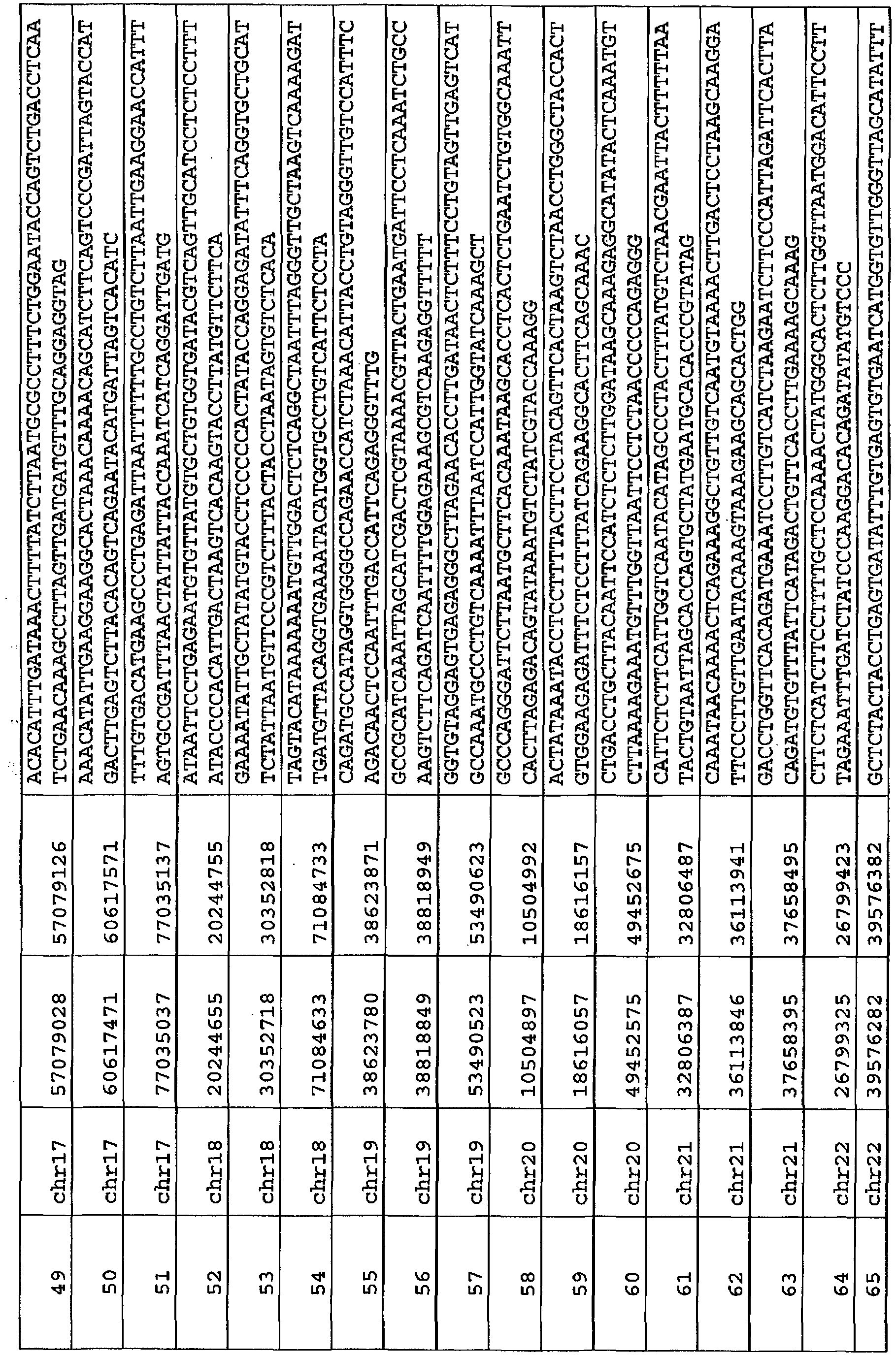

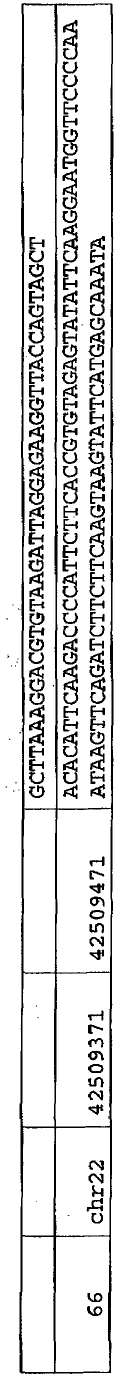

The sequences shown in Table 1 are sequences that can be used as invariant controls for each of the non-sex chromosomes. Of the nucleic acid sequences shown above in Table 1, SEQ ID NOs: 2, 5, 7, 12, 13, 17, 19, 24, 25, 28, 32, 36, 38, 40, 44, 46, 50, 52, 56, 58, 62 and 66 are the preferred sequence for each corresponding chromosome. Of these sequences, SEQ ID NOs: 2, 5, 13, 19, 28, 46, 50, 56, 58 and 66 are particularly preferred for use as invariant controls for use in sample input normalization.

Nanoreporters can be used to detect any of the sequences of SEQ ID NOs: 1-66. Nanoreporters can also be used to detect fragments of these sequences. Fragments can be between 50 and 90 nucleotides in length. Fragments can also be 51, 52,53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 nucleotides in length. The target specific regions of the nanoreporters can specifically hybridize to any of the sequences of SEQ ID NOs:1-66, any fragments thereof or any complement thereof.

The invention also provides methods of detecting the copy number of multiple genomic sequences simultaneously. Multiple probes made according to the nCounter® system of the invention can be used to detect multiple genomic targets in a multiplexed format. The signal generated by the presence of a number of copies of these targets can then be compared to a standard or reference sample with a known copy number. As explained above, a control or multiple controls are used to normalize sample DNA input between different assays.

The basis of the nCounter® Analysis system is the unique code assigned to each gene to be assayed (WO 2008/124847 and Geiss et al. Nature Biotechnology. 2008. 26(3): 317-325).

The code is composed of a number of positions (visualized as "spots") and a number of colors. Colors are chosen to minimize spectral overlap during imaging. The number of positions are chosen with respect to a combination of factors that include, but are not limited to, the length of the DNA backbone, the minimum spot size that can be resolved under current imaging conditions, flexibility in code selection for modestly-sized gene sets (*i.e*. <1000 genes) and the number of potential codes for future versions of the system (for instance, in a system with 7 positions, and 4 colors, the maximal number of specific labels is 4⁷, or 16,384 labels, if all possible combinations of codes are used).

Specific capture and reporter probes are synthesized in 96-well plates using a semi-automated process. Briefly, the reporter probes are pooled. Gene-specific probes are ligated to reporter molecule backbones and the ligated backbones were annealed to a unique pool of dye-coupled RNA or DNA segments corresponding to a single code. Reporter probes are then purified using a common 5'-repeat sequence at the end of each backbone to remove excess probe oligonucleotides and dye-coupled RNA or DNA segments. Capture probes were made by ligating a second sequence-specific oligonucleotide for each gene to a universal sequence containing biotin. After ligation, the capture probes were also pooled and affinity-purified using the universal sequence to remove the excess unligated gene-specific oligonucleotides. Reporter and capture probes were combined into a single "library" and used as a single reagent in subsequent hybridizations.

The expression levels of all DNA:reporter molecule:capture probe duplexes are measured in a single multiplexed hybridization reaction. The sample is combined with the probe library, and hybridization occurs in solution. After hybridization, the tripartite hybridized complexes are purified in a two-step procedure using magnetic beads linked to oligonucleotides complementary to universal sequences present on the capture and reporter probes. This dual purification process allows the hybridization reaction to be driven to completion with a large excess of gene-specific probes, as they were ultimately removed, and, thus, do not interfere with binding and imaging of the sample. All post hybridization steps are handled robotically on a custom liquid-handling robot (Prep Station, NanoString Technologies).

Purified reactions are deposited by the Prep Station into individual flow cells of a sample cartridge, bound to a streptavidin-coated surface via the capture probe, electrophoresed to elongate the reporter probes, and immobilized. After processing, the sample cartridge is transferred to a fully automated imaging and data collection device (Digital Analyzer, NanoString Technologies). The expression level of a gene is measured by imaging each sample in, for instance, 4 colors and counting the number of times the code for that gene is detected. For each sample, over 600 fields-of-view (FOV) are imaged (1376 X 1024 pixels) representing approximately 10 mm² of the binding surface. Typical imaging density is 100-200 counted reporters per field of view depending on the degree of multiplexing, the amount of RNA, and overall gene expression levels. However, the system is capable of operating at densities 5- to 10-fold higher. The Digital Analyzer can accommodate up to 6 cartridges at once and current scan times for 600 FOV are 4 hours per sample cartridge. Unattended, the system can process 72 samples in 24-hours per instrument.

Image processing and code counting is performed. To minimize false positives, a reporter must meet stringent criteria concerning the number, size, brightness and spacing of the spots to ensure that the code is interpreted correctly. Reporters that do not meet all of these criteria are discarded. Using these criteria, approximately 40% of the detected molecules are typically counted. No parity schemes or error correction are employed in the current system. Data is output in simple spreadsheet format listing the number of counts per DNA, or genomic DNA fragment, per sample.

### Therapeutic Applications

Kits and methods of the invention are used to detect gene or chromosome copy number in subjects who are at risk of developing an illness or disorder. Moreover, the kits and methods of the invention are used to detect gene or chromosome copy number in subjects who have been diagnosed with an illness or disorder, and who are in need of a diagnosis or prognosis. The kits and methods described herein are used to monitor disease progression (onset of a genetic disease or degeneration of telomeres as a consequence of aging or increased cell proliferation due to cancer) or responses to genetic therapy. Furthermore, the kits and methods provided herein are used to screen individuals for their personal risk of developing a disorder as well as their risk of passing a disorder onto future children. Embryonic cells are tested using the compositions and methods of the invention for the presence or absence of disorders.

The invention can be used to determine the risk of developing a particular biological condition, a particular disease, such as a cancer, a genetic disorder, a developmental disorder, a degenerative disorder, a neurological disorder, a stem cell disorder, or other biological condition. Furthermore, the present invention can be used to monitor the progression of a disease or responses to genetic therapy. Specifically, the invention can be used to detect, to monitor progression of, or monitor therapeutic regimens for diseases of the heart, kidney, ureter, bladder, urethra, liver, prostate, heart, blood vessels, bone marrow, skeletal muscle, smooth muscle, various specific regions of the brain (including, but not limited to the amygdala, caudate nucleus, cerebellum, corpus callosum, fetal, hypothalamus, thalamus), spinal cord, peripheral nerves, retina, nose, trachea, lungs, mouth, salivary gland, esophagus, stomach, small intestines, large intestines, hypothalamus, pituitary, thyroid, pancreas, adrenal glands, ovaries, oviducts, uterus, placenta, vagina, mammary glands, testes, seminal vesicles, penis, lymph nodes, thymus, and spleen. The present invention can be used to detect, to monitor progression of, or monitor therapeutic regimens for a particular disease, such as a cancer, a genetic disorder, a developmental disorder, a degenerative disorder, a neurological disorder, a stem cell disorder, or other biological condition.

Critically, the methods of the invention can determine the individual contributions of multiple genes to the same condition or disorder using one multiplexed reaction. Specifically, the methods of the invention can not only identify which genes are involved in a particular disorder, disease, or syndrome, but also by which mechanism, e.g. sequence duplication, mutation, deletion, or translocation. As such, the invention provides superior properties over all known methods of genetic screening because the methods provided elucidate the genes involved in complex multigene disorders such as Autism or Down Syndrome, which, unlike Huntington's Disease, require the participation of multiple genes. Most importantly, the methods of the invention do not require the skilled artisan to have identified or predetermined which genes may contribute to a particular disorder, disease, or syndrome prior to using these methods. If the skilled artisan applies gene specific probes that are specific for all known genes, then the abnormally increased or decreased genomic DNA copy numbers present in a sample taken from a subject having a particular disorder, disease, or syndrome will be apparent when compared to the genomic DNA copy numbers of a normal subject.

In certain embodiments of these methods, the term "normal subjects" is meant to describe a person who has not been diagnosed with the disorder, disease, or syndrome under examination. Alternatively, a "normal subject" is a person of similar age, weight, gender, ethnicity and physical health who has not been diagnosed with the particular disorder, disease, or syndrome under examination. In other aspects, a "normal subject" is a person who has not been diagnosed with any genetic disorder, disease, or syndrome, and furthermore, may be of similar age, weight, gender, ethnicity and physical health to the test subject. In another aspect, a normal subject is a predetermined numerical reference based upon, for example, national or international averages or standards.

The methods of the invention encompass a variety of subjects. Subjects are plants or animals. Animals are mammals. In certain embodiments, the mammal is a human, non-human primate, mouse, rat, dog, cat, horse, or cow, but is not limited to these examples. Mammals other than humans are advantageously used as subjects that represent animal models of a particular disorder. The preferred subject is human.

The methods of the invention also encompass screening of subjects for pathology at various points in their lives. In certain embodiments, the detection is performed prenatally, neonatally, postnatally, at infancy, childhood, puberty, early adulthood, adulthood, and during old age. The pre-natal subject may be tested at about 1, 2, 3, 4, 5, 6, 7, 8 or 9 months prior to their expected birth date. Other subjects may be tested at the age of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, and 99 years of age or older.

### Cancer

Kits and methods of the invention are used to identify cells and subjects at risk of developing or those cells and subjects who may have a predisposition for developing cancer. Moreover, the kits and methods of the invention are used to differentiate cancer cell type, cancer subtype, tumor grade, or cancer stage for the purpose of diagnosing or prognosing a subject at risk of developing cancer or a subject who has developed cancer. The kits and methods of the invention are further used to monitor to progression of a tumor, cancer, or a treatment regime. Additionally, the kits and methods of the invention are used to screen individuals for any genetic predisposition to developing cancer.

The term "cancer" includes solid tumors, as well as, hematologic tumors and/or malignancies. A "precancer cell" or "precancerous cell" is a cell manifesting a cell proliferative disorder that is a precancer or a precancerous condition. A "cancer cell" or "cancerous cell" is a cell manifesting a cell proliferative disorder that is a cancer. Any reproducible means of measurement may be used to identify cancer cells or precancerous cells. Cancer cells or precancerous cells can be identified by histological typing or grading of a tissue sample (*e.g*., a biopsy sample). Cancer cells or precancerous cells can be identified through the use of appropriate molecular markers.

The kits and methods of the invention are used to further determine cancer severity, as it is characterized by stage, tumor grade, and expression of factors that degrade the extracellular matrix, induce vascularization, inhibit cell adhesion and enable metastasis.

Exemplary cancers include, but are not limited to, adrenocortical carcinoma, AIDS-related cancers, AIDS-related lymphoma, anal cancer, anorectal cancer, cancer of the anal canal, appendix cancer, childhood cerebellar astrocytoma, childhood cerebral astrocytoma, basal cell carcinoma, skin cancer (non-melanoma), biliary cancer, extrahepatic bile duct cancer, intrahepatic bile duct cancer, bladder cancer, uringary bladder cancer, bone and joint cancer, osteosarcoma and malignant fibrous histiocytoma, brain cancer, brain tumor, brain stem glioma, cerebellar astrocytoma, cerebral astrocytoma/malignant glioma, ependymoma, medulloblastoma, supratentorial primitive neuroectodeimal tumors, visual pathway and hypothalamic glioma, breast cancer, bronchial adenomas/carcinoids, carcinoid tumor, gastrointestinal, nervous system cancer, nervous system lymphoma, central nervous system cancer, central nervous system lymphoma, cervical cancer, childhood cancers, chronic lymphocytic leukemia, chronic myelogenous leukemia, chronic myeloproliferative disorders, colon cancer, colorectal cancer, cutaneous T-cell lymphoma, lymphoid neoplasm, mycosis fungoides, Seziary Syndrome, endometrial cancer, esophageal cancer, extracranial germ cell tumor, extragonadal germ cell tumor, extrahepatic bile duct cancer, eye cancer, intraocular melanoma, retinoblastoma, gallbladder cancer, gastric (stomach) cancer, gastrointestinal carcinoid tumor, gastrointestinal stromal tumor (GIST), germ cell tumor, ovarian germ cell tumor, gestational trophoblastic tumor glioma, head and neck cancer, hepatocellular (liver) cancer, Hodgkin lymphoma, hypopharyngeal cancer, intraocular melanoma, ocular cancer, islet cell tumors (endocrine pancreas), Kaposi Sarcoma, kidney cancer, renal cancer, kidney cancer, laryngeal cancer, acute lymphoblastic leukemia, acute myeloid leukemia, chronic lymphocytic leukemia, chronic myelogenous leukemia, hairy cell leukemia, lip and oral cavity cancer, liver cancer, lung cancer, non-small cell lung cancer, small cell lung cancer, AIDS-related lymphoma, non-Hodgkin lymphoma, primary central nervous system lymphoma, Waldenstram macroglobulinemia, medulloblastoma, melanoma, intraocular (eye) melanoma, merkel cell carcinoma, mesothelioma malignant, mesothelioma, metastatic squamous neck cancer, mouth cancer, cancer of the tongue, multiple endocrine neoplasia syndrome, mycosis fungoides, myelodysplastic syndromes, myclodysplastic/ myeloproliferative diseases, chronic myelogenous leukemia, acute myeloid leukemia, multiple myeloma, chronic myeloproliferative disorders, nasopharyngeal cancer, neuroblastoma, oral cancer, oral cavity cancer, oropharyngeal cancer, ovarian cancer, ovarian epithelial cancer, ovarian low malignant potential tumor, pancreatic cancer, islet cell pancreatic cancer, paranasal sinus and nasal cavity cancer, parathyroid cancer, penile cancer, pharyngeal cancer, pheochromocytoma, pineoblastoma and supratentorial primitive neuroectodermal tumors, pituitary tumor, plasma cell neoplasm/multiple myeloma, pleuropulmonary blastoma, prostate cancer, rectal cancer, renal pelvis and ureter, transitional cell cancer, retinoblastoma, rhabdomyosarcoma, salivary gland cancer, ewing family of sarcoma tumors, Kaposi Sarcoma, soft tissue sarcoma, uterine cancer, uterine sarcoma, skin cancer (non-melanoma), skin cancer (melanoma), merkel cell skin carcinoma, small intestine cancer, soft tissue sarcoma, squamous cell carcinoma, stomach (gastric) cancer, supratentorial primitive neuroectodermal tumors, testicular cancer, throat cancer, thymoma, thymoma and thymic carcinoma, thyroid cancer, transitional cell cancer of the renal pelvis and ureter and other urinary organs, gestational trophoblastic tumor, urethral cancer, endometrial uterine cancer, uterine sarcoma, uterine corpus cancer, vaginal cancer, vulvar cancer, and Wilm's Tumor.

### Developmental and Degenerative Disorders

Kits and methods of the invention are used to identify cells and subjects at risk of developing a developmental or degenerative disorder or those cells and subjects who may have a predisposition for developing a developmental or degenerative disorder. Moreover, the kits and methods of the invention are used to differentiate developmental disorders, degenerative disorders, or developmental from degenerative disorders for the purpose of diagnosing or prognosing a subject at risk of presenting or a subject who has been diagnosed with a developemental or degenerative disorder. The kits and methods of the invention are further used to monitor to progression of a developmental disorder, a degenerative disorder, or a treatment regime. Additionally, the kits and methods of the invention are used to screen individuals for any genetic predisposition for presenting a developmental or degenerative disorder himself/herself, or for producing a child having a developmental or degenerative disorder.

The term "developmental disorder" includes any disorder that initially presents in an individual during gestation or early postnatal development. Early postnatal development encompasses a period of time from birth to age 18. Although developmental disorders are often considered synonymous with mental disabilities that cause mental, emotional, or cognitive deficits, the term "developmental disorder" is meant to encompass any disorder that presents in either a fetus or a child aged 18 years or less, regardless of the specific signs or symptoms associated with the disorder. Moreover, developmental disorders are typically characterized by an inadequate or malfunctioning development of biological or psychological process. Developmental disorders are also characterized by behavioral traits, family history, brain morphology, or genetic/biomarkers that are present during development and predict or indicate the individual's risk of developing the disease in adulthood (e.g. Huntington's Disease, Amyotrophic lateral sclerosis or ALS, and Schizophrenia).

A specific developmental disorder selectively affects one area of development, sparing essentially all other areas of development. Specific developmental disorders affect primarily hearing, vision, speech, or metabolism. However, a pervasive developmental disorder involves delays in the development of many basic skills, most notably the ability to socialize with others, because these conditions affect the child's ability to communicate and to use imagination. Pervasive developmental disorders include, but are not limited to, austism and autism spectrum disorders, Asperger's syndrome, childhood disintegrative disorder, Rett's Syndrome, attention-deficit disorder (ADD), and unspecified but pervasive disorders.

Exemplary developmental disorders also include, but are not limited to, Autism spectrum disorders (ASD), Angelman Syndrome, central auditory processing disorder (CAPD), cerebral palsy, Down Sydrome, expressive language disorder, IsoDendric 15 (abbreviated idic(15)), Lanau-Kleffner Syndrome, neural tube defects, phenylketonuria (PKU), Prader-Willi Syndrome, seizure disorders, epilepsy, Tourette Syndrome, Williams Syndrome, hearing loss, deafness, blindness, vision impairment, jaundice / kernicterus, cluttering (speech disfluency), agnosias (visual, auditory, and somatosensory), anorexia nervosa disorder, acute stress disorder, adjustment disorder, bipolar disorder, body dysmorphic disorder, breathing-related sleep disorders, asthma, brief psychotic episode, bulimia nervosa, schizophrenia, Huntington's Disease (HD), multiple sclerosis (MS), amyotrophic lateral sclerosis (ALS), chronic motor or vocal tic disorder, circadian rhythm sleep disorder, conduct disorder, communication/language disorders, Cornelia de Lange Syndrome, fatal familial insomnia (FFI), Fahr's Syndrome (or idiopathic basal ganglia calcification), migraine, neoplasm (benign and malignant), Lupus erythematosus, autoimmune disorders, diabetes (type I), Wilson's Disease, Bell's Palsy, congenital heart disease, microcephaly, neonatal encephalitis, hydrocephalis, Parkinson's Disease, narcolepsy, muscular distrophy, Guillain-Barre Syndrome, neurofibromatosis, Von Hippel-Lindau Disease, dyslexia, familial hypercholesterolemia, polycystic kidney disease, hereditary spherocytosis, hereditary breast and ovarian syndrome, marfan syndrome, sickle cell anemia, sickle cell disease, cystic fibrosis, mucopolysaccharidoses, glycogen storage diseases, glactosemia, hemophilia, Androgenetic alopecia, Lebner's hereditary optic neuropathy, autoimmune disease, cleft palate, obesity, Gauchers Disease, Rett Syndrome, ataxia telagiectasia, long QT Syndrome, Alport Syndrome, male pattern baldness, SRY sex determination, achondroplasia, Cockayne syndrome, DiGeorge syndrome, fragile X syndrome, severe combined immunodeficiency, Waardenburg syndrome, Werner syndrome, Zellweger syndrome, adrenoleukodystrophy, glucose galactose malabsorption, hereditary hemochromatosis, Lesch-Nyhan syndrome, maple syrup urine disease, Menkes syndrome, Neimann-Pick syndrome, porphyria, Refsum disease, Tangier disease, Tay-Sachs disease, diastropic dysplasia, Ellis-van Creveld Syndrome (chondroectodermal dysplasia), paroxysmal nocturnal hemoglobinuria, thalassemia, Crohn's disease, Best disease, glaucoma, retinoblastoma, congenital adrenal hyperplasia, autoimmune polyglandular syndrome, multiple endocrine neoplasia, familial Mediterranean fever, immunodeficiency with hyper- IgM, Charcot-Marie-Tooth syndrome, fibrodysplasia ossificans progressive, myotonic dystrophy, essential tremor, Friedrich's Ataxia, spinal muscular atrophy, spinocerebellar ataxia, tuberous sclerosis, alpha-1-antitrypsin deficiency, and Pendred Syndrome.

The term "degenerative disorder" includes any disorder that initially presents in an adult individual. The term adult encompasses a period of time from age 18 to death. Although degenerative disorders are often considered synonymous with mental disabilities that cause mental, emotional, or cognitive deficits, the term "degenerative disorder" is meant to encompass any disorder that presents in an adult aged 18 years or older, regardless of the specific signs or symptoms associated with the disorder. Moreover, degenerative disorders are typically characterized by the deregulation or malfunction of an ordinarly operable biological or psychological process. Degenerative disorders can result from genetic predisposition, environmental factors, or exposure to pathogens such as a virus or prion.

Exemplary degenerative disorders include, but are not limited to, Alzheimer's Disease, dementia, senility, agnosias (visual, auditory, and somatosensory), acute stress disorder, adjustment disorder, bipolar disorder, body dysmorphic disorder, breathing-related sleep disorders (sleep apnea), brief psychotic episode, bulimia nervosa, schizophrenia, Huntington's Disease (HD), Parkinson's Disease, multiple sclerosis (MS), amyotrophic lateral sclerosis (ALS), Capgras (delusion) Syndrome, chronic fatique syndrome, circadian rhythm sleep disorder, conduct disorder, communication/language disorders, Creutzfeldt-Jakob Disease (CJD), kuru, Gerstmann-Sträussler-Scheinker syndrome (GSS), fatal familial insomnia (FFI), cyclothymic disorder, acquired immune deficiency syndrome (AIDS), depression, addiction, Cushing's Syndrome (also called hyperadrenocorticism or hypercorticism), neoplasm (benign and malignant), stroke, diabetes (Type II), aneurysm, cardiovascular disease (including heart disease), Meniere's Disease, deafness, blindness, multiple system atrophy, Neiman Pick Disease, artherosclerosis, progressive supranuclear palsy, cancer, Tay-Sachs Disease, keratoconus, macular degeneration, inflammatory bowel disease (IBD), prostatis, male pattern baldness, obesity, paroxysmal nocturnal hemoglobinuria, thalassemia, Crohn's disease, Best disease, glaucoma, Gyrate atrophy of the choroid and retina, Charcot-Marie-Tooth syndrome, fibrodysplasia ossificans progressive, myotonic dystrophy,osteoarthritis, osteoporosis, arthritis, and rheumatoid arthritis.

### Neurological Disorders

Kits and methods of the invention are used to identify cells and subjects at risk of developing a neurological disorder or those cells and subjects who may have a predisposition for developing a neurological disorder. Moreover, the kits and methods of the invention are used to differentiate neurological disorders for the purpose of diagnosing or prognosing a subject at risk of presenting or a subject who has been diagnosed with a neurological disorder. The kits and methods of the invention are further used to monitor to progression of a neurological disorder or a treatment regime. Additionally, the kits and methods of the invention are used to screen individuals for any genetic predisposition for presenting a neurological disorder himself/herself, or for producing a child having a neurological disorder.

The term "neurological disorder" includes any disorder that initially presents within the nervous system of an individual. Neurological disorders present with a variety of signs and symptoms including, but not limited to, psychological, mood, or behaviorial changes; loss or decreased accuity of one or more senses (vision, hearing, touch); increased pain or burning sensations; lack of coordination or balance; loss of memory; loss of control over voluntary or involuntary movement; speech or balance; visual or auditory hallucinations; seizures; headaches; decreased movement; and ultimately, coma or death. Neurological disorders can result from genetic predisposition for developing the neurological disorder, one or more environmental factors that induce a the disorder to enhance the individual's genetic predisposition, or exposure of an individual to infectious agents such as a virus, a bacteria, a fungus, or a prion that induces the disorder or enhances the individual's genetic predisposition.

Exemplary neurological disorders include, but are not limited to, autism spectrum disorders (ASD), Angelman Syndrome, bipolar disorder, attention-deficit disorder (ADD), central auditory processing disorder (CAPD), cerebral palsy, Down Sydrome, expressive language disorder, IsoDendric 15 (abbreviated idic(15)), Lanau-Kleffner Syndrome, neural tube defects, seizure disorders, epilepsy, Tourette Syndrome, traumatic brain injury (TBI), childhood disintegrative disorder, agnosias (visual, auditory, and somatosensory), anorexia nervosa disorder, acute stress disorder, adjustment disorder, bipolar disorder, body dysmorphic disorder, breathing-related sleep disorders, brief psychotic episode, bulimia nervosa, schizophrenia, Huntington's Disease (HD), multiple sclerosis (MS), amyotrophic lateral sclerosis (ALS), Capgras (delusion) Syndrome, chronic motor or vocal tic disorder, circadian rhythm sleep disorder, cluttering (speech disfluency), conduct disorder, communication/language disorders, Creutzfeldt-Jakob Disease (CJD), kuru, Gerstmann-Sträussler-Scheinker syndrome (GSS), fatal familial insomnia (FFI), depression, addiction, Fahr's Syndrome (or idiopathic basal ganglia calcification), migraine, neoplasm (benign and malignant), aphasia, paralysis, Bell's Palsy, cerebrovascular disease, encephalitis, hydrocephalis, microcephaly, Parkinson's Disease, trigeminal neuralgia, narcolepsy, muscular distrophy, Guillain-Barre Syndrome, neurofibromatosis, dyslexia, Rett Syndrome, Fragile X syndrome, adrenoleukodystrophy, ataxia telangiectasia, Cockayne syndrome, deafness, Duchenne muscular dystrophy, Gaucher disease, Lesch-Nyhan syndrome, maple syrup urine disease, Menkes syndrome, phenylketonuria, Prader-Willi syndrome, spinal muscular atrophy, spinocerebellar ataxia, tuberous sclerosis, Neimann-Pick syndrome, Refsum disease, Tay-Sachs disease, Charcot-Marie-Tooth syndrome, fibrodysplasia ossificans progressive, myotonic dystrophy, and Meniere's Disease.

### Stem Cell Disorders

Kits and methods of the invention are used to identify cells and subjects at risk of developing a "stem cell" disorder or those cells and subjects who may have a predisposition for developing a stem cell disorder. Moreover, the kits and methods of the invention are used to differentiate stem cell disorders for the purpose of diagnosing or prognosing a subject at risk of presenting or a subject who has been diagnosed with a stem cell disorder. The kits and methods of the invention are further used to monitor to progression of a stem cell disorder or a treatment regime. Additionally, the kits and methods of the invention are used to screen individuals for any genetic predisposition for presenting a stem cell disorder himself/herself, or for producing a child having a stem cell disorder.

The term "stem cell disorder" includes any disorder that initially presents within a totipotent (or omnipotent), pluripotent, multipotent, oligopotent, or unipotent stem cell of an individual. Alternatively, or in addition, a stem cell disorder includes any disorder which can be treated or prevented by administering a composition including a stem cell to the individual. Stem cells are characterized by their ability to produce daughter cells, one of which will differentiate and the other of which will remain an undifferentiated stem cell. The potency of a stem cell relates to differentiation potential of the daughter cell that becomes committed to a particular cell fate. Specifically, the terms totipotent stem cell or omnipotent stem cell describe stem cells that can give rise to both embryonic stem cells or, alternatively, the stem cell can generate every type of cell in the human body. Pluripotent stem cells have a more restricted potential than totipotent stem cells, however, these stem cells can generate cells derived from any of the three germ layers (ectoderm, mesoderm, or endoderm). Multipotent stem cells have a more restricted potential than pluripotent stem cells, however, these stem cells can generate cells within a related lineage. Multipotent stem cells are often considered adult stem cells because they are found in, for instance, the adult brain (neural stem cells that give rise to neurons and all types of glia) and bones (bone marrow stem cells that give rise to all types of blood cells). Oligopotent stem cells have a more restricted potential than multipotent stem cells, however, these stem cells can generate a few related types of cells. For example, the corneal epithelium contains oliopotent stem cells that produce only corneal and conjunctival cells. Unipotent cells are the most restricted cell type because they can only reproduce their own cell type, however, they do maintain the ability to self-renew. Muscle stem cells are nonlimiting examples of unipotent stem cells.

Exemplary stem cell disorders include, but are not limited to, autism spectrum disorders (ASD), neural tube defects, seizure disorders, epilepsy, hearing loss, deafness, blindness, vision impairment, jaundice / kernicterus, cluttering (speech disfluency), agnosias (visual, auditory, and somatosensory), Huntington's Disease (HD), multiple sclerosis (MS), amyotrophic lateral sclerosis (ALS), chronic motor or vocal tic disorder, circadian rhythm sleep disorder, Alzheimer's Disease, dementia, senility, diabetes, Parkinson's Disease, muscular distrophy, Guillain-Barre Syndrome, sickle cell anemia or sickle cell disease, ataxia telagiectasia, Cockayne syndrome, DiGeorge syndrome, severe combined immunodeficiency, porphyria, paroxysmal nocturnal hemoglobinuria, thalassemia, familial Mediterranean fever, immunodeficiency with hyper- IgM, Charcot-Marie-Tooth syndrome, fibrodysplasia ossificans progressive, myotonic dystrophy, spinal muscular atrophy, spinocerebellar ataxia, and Gauchers Disease.

### Nanoreporters and nCounter® System overview

The basis of the nCounter® Analysis system is the unique code assigned to each nucleic acid target to be assayed (WO 2008/124847 and Geiss et al. Nature Biotechnology. 2008. 26(3): 317-325).

The code is composed of an ordered series of colored fluorescent spots which create a unique barcode for each target to be assayed. A pair of probes is designed for each DNA or RNA target, a biotinylated capture probe and a reporter probe carrying the fluorescent barcode. This system is also referred to, herein, as the nanoreporter code system.

Specific reporter and capture probes are synthesized for each target. Briefly, sequence-specific DNA oligonucleotide probes are attached to code-specific reporter molecules. Capture probes are made by ligating a second sequence-specific DNA oligonucleotide for each target to a universal oligonucleotide containing biotin. Reporter and capture probes are all pooled into a single hybridization mixture, the "probe library".

The relative abundance of each target is measured in a single multiplexed hybridization reaction. The sample is combined with the probe library, and hybridization occurs in solution. After hybridization, the tripartite hybridized complexes are purified in a two-step procedure using magnetic beads linked to oligonucleotides complementary to universal sequences present on the capture and reporter probes. This dual purification process allows the hybridization reaction to be driven to completion with a large excess of target-specific probes, as they are ultimately removed, and, thus, do not interfere with binding and imaging of the sample. All post hybridization steps are handled robotically on a custom liquid-handling robot (Prep Station, NanoString Technologies).

Purified reactions are deposited by the Prep Station into individual flow cells of a sample cartridge, bound to a streptavidin-coated surface via the capture probe, electrophoresed to elongate the reporter probes, and immobilized. After processing, the sample cartridge is transferred to a fully automated imaging and data collection device (Digital Analyzer, NanoString Technlogies). The expression level of a target is measured by imaging each sample and counting the number of times the code for that target is detected. For each sample, typically 600 fields-of-view (FOV) are imaged (1376 X 1024 pixels) representing approximately 10 mm² of the binding surface. Typical imaging density is 100-1200 counted reporters per field of view depending on the degree of multiplexing, the amount of sample input, and overall target abundance. Data is output in simple spreadsheet format listing the number of counts per target, per sample.

Many nanoreporters, referred to as singular nanoreporters, are composed of one molecular entity. However, to increase the specificity of a nanoreporter and/or to improve the kinetics of its binding to a target molecule, a preferred nanoreporter is a dual nanoreporter composed of two molecular entities, each containing a different target-specific sequence that binds to a different region of the same target molecule. In a dual nanoreporter, at least one of the two nanoreporter probes is labeled. This labeled nanoreporter probe is referred to herein as a "reporter probe". The other nanoreporter probe is not necessarily labeled. Such unlabeled components of dual nanoreporters are referred to herein as "capture probes" and often have affinity tags attached, such as biotin, which are useful to immobilize and/or stretch the complex containing the dual nanoreporter and the target molecule to allow visualization and/or imaging of the complex. When both probes are labeled or both have affinity tags, the probe with more label monomer attachment regions is referred to as the reporter probe and the other probe in the pair is referred to as a capture probe.

For both single and dual nanoreporters, a fully assembled and labeled nanoreporter probe comprises two main portions, a target-specific sequence that is capable of binding to a target molecule, and a labeled portion which provides a "code" of signals associated with the target-specific sequence. Upon binding of the nanoreporter probe to the target molecule, the code identifies the target molecule to which the nanoreporter is bound.

Nanoreporters are modular structures. In some embodiments, the nanoreporter comprises a plurality of different detectable molecules. In some embodiments, a labeled nanoreporter, is a molecular entity containing certain basic elements: (i) a plurality of unique label attachment regions attached in a particular, unique linear combination, and (ii) complementary polynucleotide sequences attached to the label attachment regions of the backbone. In some embodiments, the labeled nanoreporter comprises 2, 3, 4, 5, 6, 7, 8, 9, 10 or more unique label attachment regions attached in a particular, unique linear combination, and complementary polynucleotide sequences attached to the label attachment regions of the backbone. In some embodiments, the labeled nanoreporter comprises 6 or more unique label attachment regions attached in a particular, unique linear combination, and complementary polynucleotide sequences attached to the label attachment regions of the backbone. A nanoreporter probe further comprises a target-specific sequence, also attached to the backbone.

The term label attachment region includes a region of defined polynucleotide sequence within a given backbone that may serve as an individual attachment point for a detectable molecule. In some embodiments, the label attachment regions comprise designed sequences.

In some embodiments, the label nanoreporter also comprises a backbone containing a constant region. The term constant region includes tandemly-repeated sequences of about 10 to about 25 nucleotides that are covalently attached to a nanoreporter. The constant region can be attached at either the 5' region or the 3' region of a nanoreporter, and may be utilized for capture and immobilization of a nanoreporter for imaging or detection, such as by attaching to a solid substrate a sequence that is complementary to the constant region. In certain aspects, the constant region contains 2, 3, 4, 5, 6, 7, 8, 9, 10, or more tandemly-repeated sequences, wherein the repeat sequences each comprise about 8, 9, 10, 11, 12, 13, 14, 15, 16, 17, 18, 19, 20, 21, 22, 23, 24, 25, 26, 27, 28, 29, 30, or more nucleotides, including about 12-18, 13-17, or about 14-16 nucleotides.

The nanoreporters described herein can comprise synthetic, designed sequences. In some embodiments, the sequences contain a fairly regularly-spaced pattern of a nucleotide (*e*.*g*. adenine) residue in the backbone. In some embodiments, a specific nucleotide is spaced at least an average of 8, 9, 10, 12, 15, 16, 20, 30, or 50 bases apart. In some embodiments, a nucleotide is spaced at least an average of 8 to 16 bases apart. In some embodiments, a nucleotide is spaced at least an average of 8 bases apart. This allows for a regularly spaced complementary nucleotide in the complementary polynucleotide sequence having attached thereto a detectable molecule. For example, in some embodiments, when the nanoreporter sequences contain a fairly regularly-spaced pattern of adenine residues in the backbone, whose complement is a regularly-spaced pattern of uridine (U) residues in complementary RNA segments, the in vitro transcription of the segments can be done using an aminoallyl-modified uridine base, which allows the covalent amine coupling of dye molecules at regular intervals along the segment. In some embodiments, the sequences contain about the same number or percentage of a nucleotide (*e*.*g*. adenine) that is spaced at least an average of 8, 9, 10, 12, 15, 16, 20, 30, or 50 bases apart in the sequences. This allows for similar number or percentages in the complementary polynucleotide sequence having attached thereto a detectable molecule. Thus, in some embodiments, the sequences contain a nucleotide that is not regularly-spaced but that is spaced at least an average of 8, 9,10, 12, 15, 16, 20, 30, or 50 bases apart. In some embodiments, 20%, 30%, 50%, 60%, 70%, 80%, 90% or 100% of the complementary nucleotide is coupled to a detectable molecule. For instance, in some embodiments, when the nanoreporter sequences contain a similar percentage of adenine residues in the backbone and the in vitro transcription of the complementary segments is done using an aminoallyl-modified uridine base, 20%, 30%, 50%, 60%, 70%, 80%, 90% or 100% of the aminoallyl-modified uridine base can be coupled to a detectable molecule. Alternatively, the ratio of aminoallyl-modified uridine bases and uridine bases can be changed during the in vitro transcription process to achieve the desired number of sites which can be attached to a detectable molecule. For example, in vitro transcription process can take place in the presence of a mixture with a ratio of 1/1 of uridine to aminoallyl-modified uridine bases, when some or all the aminoallyl-modified uridine bases can be coupled to a detectable molecule.

In some embodiments, the nanoreporters described herein have a fairly consistent melting temperature (Tm). Without intending to be limited to any theory, the Tm of the nanoreporters described herein provides for strong bonds between the nanoreporter backbone and the complementary polynucleotide sequence having attached thereto a detectable molecule, therefore, preventing dissociation during synthesis and hybridization procedures. In addition, the consistent Tm among a population of nanoreporters allows for the synthesis and hybridization procedures to be tightly optimized, as the optimal conditions are the same for all spots and positions. In some embodiments, the sequences of the nanoreporters have a 50% guanine/cytosine (G/C), with no more than three G's in a row. Thus, in some embodiments, the disclosure provides a population of nanoreporters in which the Tm among the nanoreporters in the population is fairly consistent. In some embodiments, the disclosure provides a population of nanoreporters in which the Tm of the complementary polynucleotide sequences when hybridized to its label attachment regions is about 80° C, 85° C, 90° C, 100° C or higher. In some embodiments, the disclosure provides a population of nanoreporters in which the Tm of the complementary polynucleotide sequences when hybridized to its label attachment regions is about 80° C or higher.

In some embodiments, the nanoreporters described herein have minimal or no secondary structures, such as any stable intra-molecular base-paring interaction (*e*.*g*. hairpins). Without intending to be limited to any theory, the minimal secondary structure in the nanoreporters provides for better hybridization between the nanoreporter backbone and the polynucleotide sequence having attached thereto a detectable molecule. In addition, the minimal secondary structure in the nanoreporters provides for better detection of the detectable molecules in the nanoreporters. In some embodiments, the nanoreporters described herein have no significant intra-molecular pairing under annealing conditions of 75° C, 1 X SSPE. Secondary structures can be predicted by programs known in the art such as MFOLD. In some embodiments, the nanoreporters described herein contain less than 1% of inverted repeats in each strand, wherein the inverted repeats are 9 bases or greater. In some embodiments, the nanoreporters described herein contain no inverted repeats in each strand. In some embodiments, the nanoreporters do not contain any inverted repeat of 9 nucleotides or greater across a sequence that is 1100 base pairs in length. In some embodiments, the nanoreporters do not contain any inverted repeat of 7 nucleotides or greater across any 100 base pair region. In some embodiments, the nanoreporters described herein contain less than 1% of inverted repeats in each strand, wherein the inverted repeats are 9 nucleotides or greater across a sequence that 1100 base pairs in length. In some embodiments, the nanoreporters described herein contain less than 1% of inverted repeats in each strand, wherein the inverted repeats are 7 nucleotides or greater across any 100 base pair region. In some embodiments, the nanoreporters described herein contain a skewed strand specific content such that one strand is CT-rich and the other is GA-rich.

The disclosure also provides unique nanoreporters. In some embodiments, the nanoreporters described herein contain less that 1% of direct repeats. In some embodiments, the nanoreporters described herein contain no direct repeats. In some embodiments, the nanoreporters do not contain any direct repeat of 9 nucleotides or greater across a sequence that 1100 base pairs in length. In some embodiments, the labeled nanoreporters do not contain any direct repeat of 7 nucleotides or greater across any 100 base pair region. In some embodiments, the nanoreporters described herein contain less than 1% of direct repeats in each strand, wherein the direct repeats are 9 nucleotides or greater across a sequence that 1100 base pairs in length. In some embodiments, the nanoreporters described herein contain less than 1% of direct repeats in each strand, wherein the direct repeats are 7 nucleotides or greater across any 100 base pair region. In some embodiments, the nanoreporters described herein contain less than 85, 80, 70, 60, 50, 40, 30, 20, 10, or 5% homology to any other sequence used in the backbones or to any sequence described in the REFSEQ public database. In some embodiments, the nanoreporters described herein contain less than 85% homology to any other sequence used in the backbones or to any sequence described in the REFSEQ public database. In some embodiments, the nanoreporters described herein contain less than 20, 16, 15, 10, 9, 7, 5, 3, 2 contiguous bases of homology to any other sequence used in the backbones or to any sequence described in the REFSEQ public database. In some embodiments, the nanoreporters described herein have no more than 15 contiguous bases of homology and no more than 85% identity across the entire length of the nanoreporter to any other sequence used in the backbones or to any sequence described in the REFSEQ public database.

In some embodiments, the sequence characteristics of the nanoreporter probes described herein provide sensitive detection of a target molecule. For instance, the binding of the nanoreporter probes to target molecules which results in the identification of the target molecules can be performed by individually detecting the presence of the nanoreporter. This can be performed by individually counting the presence of one or more of the nanoreporter molecules in a sample.

The complementary polynucleotide sequences attached to a nanoreporter backbone serve to attach detectable molecules, or label monomers, to the nanoreporter backbone. The complementary polynucleotide sequences may be directly labeled, for example, by covalent incorporation of one or more detectable molecules into the complementary polynucleotide sequence. Alternatively, the complementary polynucleotide sequences may be indirectly labeled, such as by incorporation of biotin or other molecule capable of a specific ligand interaction into the complementary polynucleotide sequence. In such instances, the ligand (*e*.*g*., streptavidin in the case of biotin incorporation into the complementary polynucleotide sequence) may be covalently attached to the detectable molecule. Where the detectable molecules attached to a label attachment region are not directly incorporated into the complementary polynucleotide sequence, this sequence serves as a bridge between the detectable molecule and the label attachment region, and may be referred to as a bridging molecule, *e*.*g*., a bridging nucleic acid.

The nucleic-acid based nanoreporter and nanoreporter-target complexes described herein comprise nucleic acids, which may be affinity-purified or immobilized using a nucleic acid, such as an oligonucleotide, that is complementary to the constant region or the nanoreporter or target nucleic acid. As noted above, in some embodiments the nanoreporters comprise at least one constant region, which may serve as an affinity tag for purification and/or for immobilization (for example to a solid surface). The constant region typically comprises two or more tandemly-repeated regions of repeat nucleotides, such as a series of 15-base repeats. In such exemplary embodiments, the nanoreporter, whether complexed to a target molecule or otherwise, can be purified or immobilized by an affinity reagent coated with a 15-base oligonucleotide which is the reverse complement of the repeat unit.

Nanoreporters, or nanoreporter-target molecule complexes, can be purified in two or more affinity selection steps. For example, in a dual nanoreporter, one probe can comprise a first affinity tag and the other probe can comprise a second (different) affinity tag. The probes are mixed with target molecules, and complexes comprising the two probes of the dual nanoreporter are separated from unbound materials (*e*.*g*., the target or the individual probes of the nanoreporter) by affinity purification against one or both individual affinity tags. In the first step, the mixture can be bound to an affinity reagent for the first affinity tag, so that only probes comprising the first affinity tag and the desired complexes are purified. The bound materials are released from the first affinity reagent and optionally bound to an affinity reagent for the second affinity tag, allowing the separation of complexes from probes comprising the first affinity tag. At this point only full complexes would be bound. The complexes are finally released from the affinity reagent for the second affinity tag and then preferably stretched and imaged. The affinity reagent can be any solid surface coated with a binding partner for the affinity tag, such as a column, bead (*e.g*., latex or magnetic bead) or slide coated with the binding partner. Immobilizing and stretching nanoreporters using affinity reagents is fully described in U.S. Publication No. 2010/0161026.

The sequence of signals provided by the label monomers associated with the various label attachment regions of the backbone of a given nanoreporter allows for the unique identification of the nanoreporter. For example, when using fluorescent labels, a nanoreporter having a unique identity or unique spectral signature is associated with a target-specific sequence that recognizes a specific target molecule or a portion thereof. When a nanoreporter is exposed to a mixture containing the target molecule under conditions that permit binding of the target-specific sequence(s) of the nanoreporter to the target molecule, the target-specific sequence(s) preferentially bind(s) to the target molecule. Detection of the nanoreporter signal, such as the spectral code of a fluorescently labeled nanoreporter, associated with the nanoreporter allows detection of the presence of the target molecule in the mixture (qualitative analysis). Counting all the label monomers associated with a given spectral code or signature allows the counting of all the molecules in the mixture associated with the target-specific sequence coupled to the nanoreporter (quantitative analysis). Nanoreporters are thus useful for the diagnosis or prognosis of different biological states (*e.g*., disease vs. healthy) by quantitative analysis of known biological markers or copy number variant loci.

Many nanoreporters, referred to as singular nanoreporters, are composed of one molecular entity. However, to increase the specificity of a nanoreporter and/or to improve the kinetics of its binding to a target molecule, a nanoreporter can be a dual nanoreporter composed of two molecular entities, each containing a different target-specific sequence that binds to a different region of the same target molecule. In a dual nanoreporter, at least one of the two molecular entities is labeled. The other molecular entity need not necessarily be labeled. Such unlabeled components of dual nanoreporters may be used as capture probes and optionally have affinity tags attached, such as biotin, which are useful to immobilize and/or stretch the complex containing the dual nanoreporter and the target molecule to allow visualization and/or imaging of the complex. For instance, in some embodiments, a dual nanoreporter with a 6-position nanoreporter code, uses one 6-position coded nanoreporter (also referred to herein as a reporter probe) and a capture probe. In some embodiments, a dual nanoreporter with a 6-position nanoreporter code can be used, using one capture probe with an affinity tag and one 6-position nanoreporter component. In some embodiments an affinity tag is optionally included and can be used to purify the nanoreporter or to immobilize the nanoreporter (or nanoreporter-target molecule complex) for the purpose of imaging.

In some embodiments, the nucleotide sequences of the individual label attachment regions within each nanoreporter are different from the nucleotide sequences of the other label attachment regions within that nanoreporter, preventing rearrangements, such recombination, sharing or swapping of the label polynucleotide sequences. The number of label attachment regions to be formed on a backbone is based on the length and nature of the backbone, the means of labeling the nanoreporter, as well as the type of label monomers providing a signal to be attached to the label attachment regions of the backbone. In some embodiments, the complementary nucleotide sequence of each label attachment region is assigned a specific detectable molecule.

The disclosure also provides labeled nanoreporters wherein one or more label attachment regions are attached to a corresponding detectable molecule, each detectable molecule providing a signal. For example, in some embodiments, a labeled nanoreporter according to the disclosure is obtained when at least three detectable molecules are attached to three corresponding label attachment regions of the backbone such that these labeled label attachment regions, or spots, are distinguishable based on their unique linear arrangement. A "spot," in the context of nanoreporter detection, is the aggregate signal detected from the label monomers attached to a single label attachment site on a nanoreporter, and which, depending on the size of the label attachment region and the nature (*e*.*g*., primary emission wavelength) of the label monomer, may appear as a single point source of light when visualized under a microscope. Spots from a nanoreporter may be overlapping or non-overlapping. The nanoreporter code that identifies that target molecule can comprise any permutation of the length of a spot, its position relative to other spots, and/or the nature (*e*.*g*., primary emission wavelength(s)) of its signal. Generally, for each probe or probe pair described herein, adjacent label attachment regions are non-overlapping, and/or the spots from adjacent label attachment regions are spatially and/or spectrally distinguishable, at least under the detection conditions (*e.g*., when the nanoreporter is immobilized, stretched and observed under a microscope, as described in U.S. Publication No. 2010/0112710.

Occasionally, reference is made to a spot size as a certain number of bases or nucleotides. As would be readily understood by one of skill in the art, this refers to the number of bases or nucleotides in the corresponding label attachment region.

The order and nature (*e*.*g*., primary emission wavelength(s), optionally also length) of spots from a nanoreporter serve as a nanoreporter code that identifies the target molecule capable of being bound by the nanoreporter through the nanoreporter's target specific sequence(s). When the nanoreporter is bound to a target molecule, the nanoreporter code also identifies the target molecule. Optionally, the length of a spot can be a component of the nanoreporter code.

Detectable molecules providing a signal associated with different label attachment regions of the backbone can provide signals that are indistinguishable under the detections conditions ("like" signals), or can provide signals that are distinguishable, at least under the detection conditions (*e.g*., when the nanoreporter is immobilized, stretched and observed under a microscope).

The disclosure also provides a nanoreporter wherein two or more detectable molecules are attached to a label attachment region. The signal provided by the detectable molecules associated with said label attachment region produces an aggregate signal that is detected. The aggregate signal produced may be made up of like signals or made up of at least two distinguishable signals (*e.g*., spectrally distinguishable signals).

In one embodiment, a nanoreporter includes at least three detectable molecules providing like signals attached to three corresponding label attachment regions of the backbone and said three detectable molecules are spatially distinguishable. In another embodiment, a nanoreporter includes at least three detectable molecules providing three distinguishable signals attached to three neighboring label attachment regions, for example three adjacent label attachment regions, whereby said at least three label monomers are spectrally distinguishable.

In other embodiments, a nanoreporter includes spots providing like or unlike signals separated by a spacer region, whereby interposing the spacer region allows the generation of dark spots, which expand the possible combination of uniquely detectable signals. The term "dark spot" refers to a lack of signal from a label attachment site on a nanoreporter. Dark spots can be incorporated into the nanoreporter code to add more coding permutations and generate greater nanoreporter diversity in a nanoreporter population. In one embodiment, the spacer regions have a length determined by the resolution of an instrument employed in detecting the nanoreporter.

In other embodiments, a nanoreporter includes one or more "double spots." Each double spot contains two or more (*e*.*g*., three, four or five) adjacent spots that provide like signals without being separated by a spacer region. Double spots can be identified by their sizes.

A detectable molecule providing a signal described herein may be attached covalently or non-covalently (*e*.*g*., via hybridization) to a complementary polynucleotide sequence that is attached to the label attachment region. The label monomers may also be attached indirectly to the complementary polynucleotide sequence, such as by being covalently attached to a ligand molecule (*e.g*., streptavidin) that is attached through its interaction with a molecule incorporated into the complementary polynucleotide sequence (*e.g*., biotin incorporated into the complementary polynucleotide sequence), which is in turn attached via hybridization to the backbone.

A nanoreporter can also be associated with a uniquely detectable signal, such as a spectral code, determined by the sequence of signals provided by the label monomers attached (*e.g*., indirectly) to label attachment regions on the backbone of the nanoreporter, whereby detection of the signal allows identification of the nanoreporter.

In other embodiments, a nanoreporter also includes an affinity tag attached to the reporter probe backbone, such that attachment of the affinity tag to a support allows backbone stretching and resolution of signals provided by label monomers corresponding to different label attachment regions on the backbone. Nanoreporter stretching may involve any stretching means known in the art including but not limited to, means involving physical, hydrodynamic or electrical means. The affinity tag may comprise a constant region.

In other embodiments, a nanoreporter also includes a target-specific sequence coupled to the backbone. The target-specific sequence is selected to allow the nanoreporter to recognize, bind or attach to a target molecule. The nanoreporters described herein are suitable for identification of target molecules of all types. For example, appropriate target-specific sequences can be coupled to the backbone of the nanoreporter to allow detection of a target molecule. Preferably the target molecule is DNA (including cDNA), RNA (including mRNA and cRNA), a peptide, a polypeptide, or a protein.

One embodiment of the disclosure provides increased flexibility in target molecule detection with label monomers described herein. In this embodiment, a dual nanoreporter comprising two different molecular entities, each with a separate target-specific region, at least one of which is labeled, bind to the same target molecule. Thus, the target-specific sequences of the two components of the dual nanoreporter bind to different portions of a selected target molecule, whereby detection of the spectral code associated with the dual nanoreporter provides detection of the selected target molecule in a biomolecular sample contacted with said dual nanoreporter.

The disclosure also provides a method of detecting the presence of a specific target molecule in a biomolecular sample comprising: (i) contacting said sample with a nanoreporter as described herein (*e*.*g*., a singular or dual nanoreporter) under conditions that allow binding of the target-specific sequences in the dual nanoreporter to the target molecule and (ii) detecting the spectral code associated with the dual nanoreporter. Depending on the nanoreporter architecture, the dual nanoreporter may be labeled before or after binding to the target molecule.

The uniqueness of each nanoreporter probe in a population of probe allows for the multiplexed analysis of a plurality of target molecules. For example, in some embodiments, each nanoreporter probe contains six label attachment regions, where each label attachment region of each backbone is different from the other label attachment regions in that same backbone. If the label attachment regions are going to be labeled with one of four colors and there are 24 possible unique sequences for the label attachment regions and each label attachment region is assigned a specific color, each label attachment region in each backbone will consist of one of four sequences. There will be 4096 possible nanoreporters in this example. The number of possible nanoreporters can be increased, for example, by increasing the number of colors, increasing the number of unique sequences for the label attachment regions and/or increasing the number of label attachment regions per backbone. Likewise the number of possible nanoreporters can be decreased by decreasing the number of colors, decreasing the number of unique sequences for the label attachment regions and/or decreasing the number of label attachment regions per backbone.

In certain embodiments, the methods of detection are performed in multiplex assays, whereby a plurality of target molecules are detected in the same assay (a single reaction mixture). In a preferred embodiment, the assay is a hybridization assay in which the plurality of target molecules are detected simultaneously. In certain embodiments, the plurality of target molecules detected in the same assay is, at least 2, at least 5 different target molecules, at least 10 different target molecules, at least 20 different target molecules, at least 50 different target molecules, at least 75 different target molecules, at least 100 different target molecules, at least 200 different target molecules, at least 500 different target molecules, or at least 750 different target molecules, or at least 1000 different target molecules. In other embodiments, the plurality of target molecules detected in the same assay is up to 50 different target molecules, up to 100 different target molecules, up to 150 different target molecules, up to 200 different target molecules, up to 300 different target molecules, up to 500 different target molecules, up to 750 different target molecules, up to 1000 different target molecules, up to 2000 different target molecules, or up to 5000 different target molecules. In yet other embodiments, the plurality of target molecules detected is any range in between the foregoing numbers of different target molecules, such as, but not limited to, from 20 to 50 different target molecules, from 50 to 200 different target molecules, from 100 to 1000 different target molecules, from 500 to 5000 different target molecules, and so on and so forth.

Additional disclosure regarding nanoreporters can be found in International Publication No. WO 07/076129 and WO 07/076132. Further, the term nucleic acid probes and nanoreporters can include the rationally designed (*e*.*g*. synthetic sequences) described in International Publication No. WO 2010/019826.

### Kits

Kits include a composition containing at least a first nanoreporter comprising a first probe and a second probe; a second nanoreporter comprising a third probe and a fourth probe; and a restriction enzyme. The first probe includes a first molecule containing a first label attachment region to which are attached one or more label monomers that emit light constituting a first signal; a second label attachment region, which is non-over-lapping with the first label attachment region, to which are attached one or more label monomers that emit light constituting a second signal; and a first target-specific sequence attached to the first molecule, wherein the target-specific sequence specifically hybridizes to a target DNA sequence. In certain embodiments of the kit, the first probe further includes an affinity tag. The first probe further includes a second target-specific sequence; and an affinity tag; wherein the first target specific sequence and the second target specific sequence specifically hybridize to the same target DNA sequence at different sites.

In other embodiments of the kit, the first molecule is a reporter probe or a capture probe. In other embodiments, the second molecule is a reporter probe or a capture probe. Reporter probes and capture probes are provided in the kit individually or in a mixture.

The restriction enzyme can be either a restriction endonuclease or a DNase. Preferably the restriction enzyme is Alu1 or Bfa1.

The kit includes a second nanoreporter comprising a third probe and a fourth probe, wherein the third probe comprises a third label attachment region to which are attached one or more label monomers that emit light constituting a third signal; a fourth label attachment region, which is non-over-lapping with the third label attachment region, to which are attached one or more label monomers that emit light constituting a fourth signal; and a third target-specific sequence attached to the third molecule, wherein the target-specific sequence specifically hybridizes to a first DNA fragment from a copy number invariant region of the genome. The fourth probe comprises a fourth target-specific sequence and a second affinity tag; wherein the third molecule and the fourth molecule specifically hybridize to different regions of the same fragment from a copy number invariant region of the genome.

In other embodiments of the kit, the third molecule is a reporter probe or a capture probe. In other embodiments, the fourth molecule is a reporter probe or a capture probe. Reporter probes and capture probes are provided in the kit individually or in a mixture.

In preferred embodiments of the kits, the labels and label monomers are fluorescent.

Kits further include a control DNA sequence of known copy number. In other embodiments, kits contain a control probe to ensure that equivalent amounts of DNA are introduced or loaded into the nCounter® system.

Kits include instructions for handling the enclosed compositions and protocols for performing singular or multiplexed fragmenting and denaturing reactions, as well as the contacting, stretching, and measuring steps described herein using the enclosed compositions. Furthermore, the instructions provide guidance for preparing the resultant tagged fragmented and hybridized genomic DNA molecule(s) for detection using the nCounter® Analysis System.

### Nucleic Acids

Also disclosed herein are isolated nucleic acid molecules that can be used as controls for genomic copy number assays. These nucleic acid molecules include the sequences shown in Table 1. Of the nucleic acid sequences shown above in Table 1, SEQ ID NOs: 2, 5, 7,12, 13, 17, 19, 24, 25, 28, 32, 36, 38, 40, 44, 46, 50, 52, 56, 58, 62 and 66 are the preferred sequence for each corresponding chromosome. Of these sequences, SEQ ID NOs: 2, 5, 13, 19, 28, 46, 50, 56, 58 and 66 are particularly preferred for use as controls.

Also disclosed are nucleic acid fragments sufficient for use as controls in genomic copy number assays. These sequences can be present in the genomic sample itself or can be added to separate control samples. Probes can also be made to detect these sequences. These probes, themselves can be nucleic acid molecules comprising any of SEQ ID NOs: 1-66 or any complement thereof. As used herein, the term "nucleic acid molecule" is intended to include DNA molecules (e.g., cDNA or genomic DNA), RNA molecules (*e*.*g*., mRNA), analogs of the DNA or RNA generated using nucleotide analogs, and derivatives, fragments and homologs thereof. The nucleic acid molecule may be single-stranded or double-stranded, but preferably is comprised double-stranded DNA.

The term "probes", as utilized herein, refers to nucleic acid sequences of variable length, preferably between at least about 10 nucleotides (nt), 100 nt, or as many as approximately, *e*.*g*., 6,000 nt, depending upon the specific use. Probes are used in the detection of identical, similar, or complementary nucleic acid sequences. In specific embodiments, these probe sequences are used to construct target specific portions of nanoreporters described above.

The term "isolated" nucleic acid molecule, as utilized herein, is one, which is separated from other nucleic acid molecules which are present in the natural source of the nucleic acid. Preferably, an "isolated" nucleic acid is free of sequences which naturally flank the nucleic acid (*i*.*e*., sequences located at the 5'- and 3'-termini of the nucleic acid) in the genomic DNA of the organism from which the nucleic acid is derived. For example, in various embodiments, the isolated nucleic acid molecules described herein can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e*.*g*., brain, heart, liver, spleen, *etc*.). Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule described herein can be isolated using standard molecular biology techniques and the sequence information provided herein. Nucleic acid molecules can be isolated using standard hybridization and cloning techniques (*e*.*g*., as described in Sambrook, et al., (eds.), MOLECULAR CLONING: A LABORATORY MANUAL 2nd Ed., Cold Spring Harbor Laboratory Press, Cold Spring Harbor, NY, 1989; and Ausubel, et al., (eds.), CURRENT PROTOCOLS IN MOLECULAR BIOLOGY, John Wiley & Sons, New York, NY, 1993.)

As described herein, an isolated nucleic acid molecule comprises a nucleic acid molecule that is a complement of the nucleotide sequence shown in SEQ ID NOs: 1-66, or a portion of this nucleotide sequence (e.g., a fragment that can be used as a probe or primer). A nucleic acid molecule that is complementary to the nucleotide sequence shown SEQ ID NOs: 1-66 is one that is sufficiently complementary that it can hydrogen bond with little or no mismatches, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Fragments provided herein are defined as sequences of at least 6 (contiguous) nucleic acids, a length sufficient to allow for specific hybridization in the case of nucleic acids and are at most some portion less than a full length sequence. Fragments may be derived from any contiguous portion of a nucleic acid or amino acid sequence of choice. Derivatives are nucleic acid sequences from the native compounds either directly or by modification or partial substitution. Analogs are nucleic acid sequences that have a structure similar to, but not identical to, the native compound but differs from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid, as described below. Derivatives or analogs of the nucleic acids of the invention include, but are not limited to, molecules comprising regions that are substantially homologous to the nucleic acids of the invention, in various embodiments, by at least about 70%, 80%, or 95% identity (with a preferred identity of 80 95%) over a nucleic acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. See *e.g.* Ausubel, et al., CURRENT PROTOCOLS IN MOLECULAR BIOLOGY, John Wiley & Sons, New York, NY, 1993, and below.

As used herein, the phrase "stringent hybridization conditions" refers to conditions under which a probe, primer or oligonucleotide will hybridize to its target sequence, but to substantially no other sequences. Stringent conditions are sequence-dependent and will be different in different circumstances. Longer sequences hybridize specifically at higher temperatures than shorter sequences.

Stringent conditions are known to those skilled in the art and can be found in Ausubel, et al., (eds.), CURRENT PROTOCOLS IN MOLECULAR BIOLOGY, John Wiley & Sons, N.Y. (1989), 6.3.1 6.3.6. Preferably, the conditions are such that sequences at least about 65%, 70%, 75%, 85%, 90%, 95%, 98%, or 99% homologous to each other typically remain hybridized to each other. A non limiting example of stringent hybridization conditions are hybridization in a high salt buffer comprising 6X SSC, 50 mM Tris HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C.

### Probe Pair Selection

Genotyping of individuals based on the relative copy number of particular genomic regions can be accomplished using a collection of probe pairs specifically designed as described below.

First, a list of genomic regions is obtained with the publically available reference genome build number, chromosome, start, and end coordinates. From this list, a custom track file is generated to be used for creation of a custom user track that can be viewed within a browser, for example, the UCSC genome browser, over the Internet.

After loading the track file into the genome browser, unmasked raw DNA sequences are downloaded using the genome table browser. This is generally downloaded as a multi-FASTA file containing all the regions sequences together, but sometimes it is downloaded as a single sequence FASTA file. The multi-FASTA file containing the region sequences is manipulated to strip extraneous characters from the header of each FASTA entry, leaving only necessary information such as region name, chromosome, start, and end positions.

Then the multi-FASTA file of region sequences is split into separate FASTA files, one file for each region. Each region file is then passed to a computer program that creates *in silico* restriction fragments of various sizes, depending on the location of restriction enzyme recognition sites in the sequence. The size of the resulting DNA fragments will vary from about 0.1 kb to about 10 kb, with an approximate average size of 0.5 kb.

When all of the regions have been processed, the resulting fragment sequences are concatenated into one big file again for loading into a database of DNA fragments. A computer program is used to read the file of region coordinates and the file of DNA fragments in order to create a data structure in the database, linking the restriction fragment sequences to the regions. When all of the fragment sequences have been loaded into the database, the Gauntlet program generates probe pairs, each probe consisting of approximately 35-50 nucleotides, specifically designed to work in the nanoString nCounter® system. Many probe pairs are created per restriction fragment during the process, with the majority failing to meet rigorous specifications for the design of matched probe pairs.

Those probes that pass the physical structure and matching criteria are loaded into the database to be further scored for fitness within the CNV assay. Every passed probe for the project is sent to a local licensed version of the BLAT program to determine the location(s) with respect to the publically available reference genome (e.g. hg18 or hg19) so that we can unambiguously map the location(s) and determine their potential for cross hybridization to other regions of the genome.

Fitness scoring is performed on the Reporter probe of each probe pair (35-50 nucleotides in length) based on: (i) the length of the restriction fragment the probe sequence is within that we know works best in the nCounter® system. This is based on a mathematical model of performance as determined by in-house experimentation. (ii) the location of the probe within the region to be analyzed. The location score is based on the number of probe pairs the customer has requested per region when they submitted them for the assay design. (iii) the results of the BLAT scoring, which gives us a measure of wether we can unambiguously map the Reporter probe within the same reference genome sequence.

The unique algorithm that was developed for fitness scoring is described in detail below. This process allows for an expansion of the number of zones (bins) per region to facilitate selection of the requested number of probe pairs evenly distributed over the entire region using the probes that will work best in the nCounter® system according to our mathematical model of fitness.

The following is the flow of our unique process of probe selection for the custom CNV assay: The user specifies the value (N) of the number of probes requested per region and the maximum value (MAX) of zones to try for an iterative processing of each region. Data is gathered from the database and arranged into data structures amenable to the process. Then, for every region, and for every iteration from N to MAX, a scoring matrix is created the exact length of the region using a natural mathematical function (absolute value of a sine wave) with the cycles specified relative to N, and increasing by the value (S) at the end of each iteration cycle.

For every Reporter probe, a fitness score is calculated using the value of the sine wave, the value of the fragment length model, and an adjusted BLAT score. As the algorithm passes through the entire region length of sequence from end to end, each probe score is tested against the highest score seen so far in the zone. If the score for the probe at that position is higher than the one saved previously, the probe is replaced with the higher scoring probe. When the algorithm has reached the end of each zone, the highest scoring probe is added to the list of probes to be included in the assay.

When the algorithm has reached the entire length of the region, the program tests if the number of probes requested (N) has been satisfied, and IF NOT, the value of N is changed to the next incremental value of N by adding S, and the process begins all over again. The cycles continue until the maximum value of zones (MAX) has been reached.

### EXAMPLES

### Example 1: Quantitation of Copy Number Variations.

Multiple probes for designed for the X (9) and Y (9) chromosomes, as well as for chromosome 18 (6) and chromosome 21(3). Cells lines were provided with known chromosomal aberrations. Samples included cells with 1-5 copies of the X chromosome, normal males and females, and cells with trisomy at chromosome 21 and 18.

Genomic DNA from the samples was digested with AluI (lhr @ 100ng/µl). 100ng of genomic DNA was used per assay. The genomic DNA was denatured prior to hybridization (5 min @ 95°C, snap cool 2 min). All experiments were performed in triplicate.

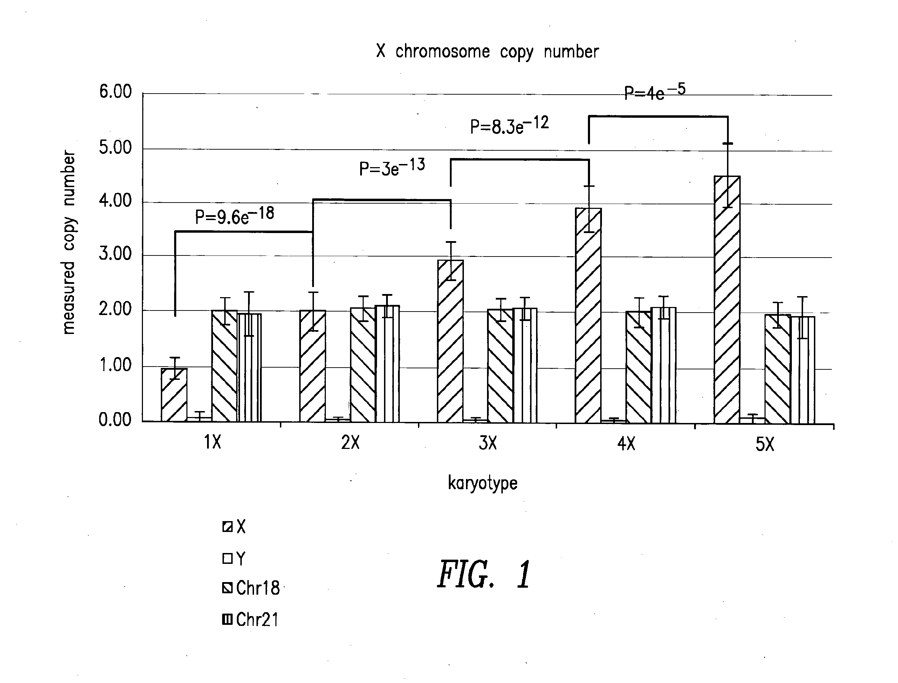

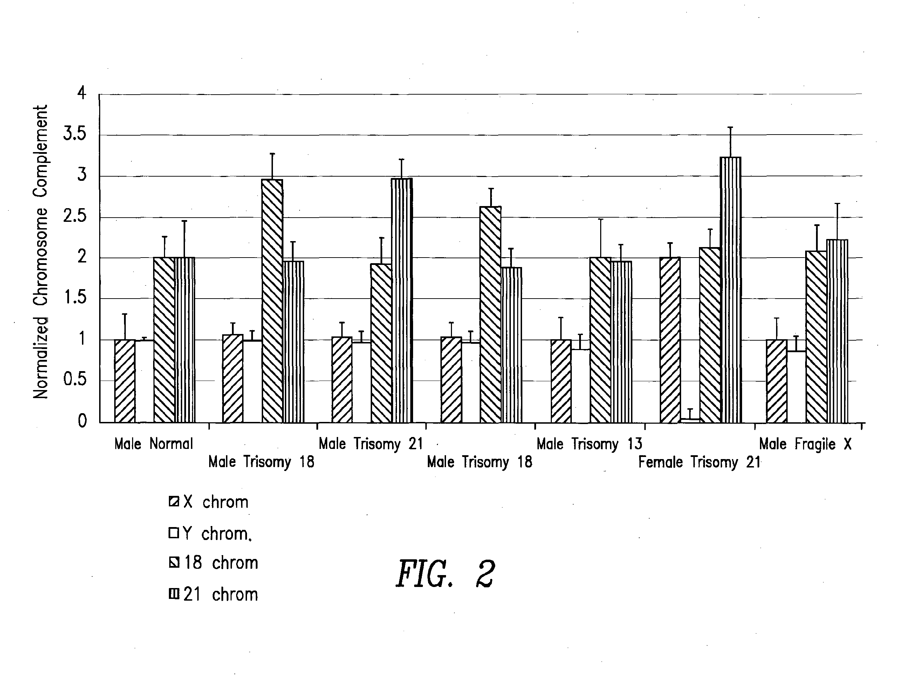

For samples with cells with 1-5 copies of the X chromosome, the samples were normalized to probes on chromosome 18 (*see* Figure 1). For trisomy detection, normalization was done to the Y chromosome in the male samples (*see* Figure 2).

The assay for multiple X chromosomes was repeated. Multi-X cell line genomic DNA purchased from Coriell cell repository. 1 µg of each sample was digested with 10U of AluI enzyme in a 50 µl digest at 37°C for 2 hours. The digested DNA was heat denatured at 95°C for 5 minutes and snap cooled on ice.

100 ng each digested/denatured sample was hybridized with the codeset CNV2 under standard nCounter® conditions for 16 hours. In addition to probes to other chromosomes, the CNV2 codeset contains 235 probes with 3 probes designed to bind to chromosome X. Standard conditions for the nCounter® assay are: 30 µl hybridization in 5X SSPE/0.1% Tween 20, 25 pM reporter probe, 100 pM capture probe, 100 ng genomic DNA, 65°C, with a heated lid for 16 hours. Raw counts were normalized to the average of 66 probes to invariant genomic regions, to correct for genome-equivalents input into each assay. Normalized counts were averaged for each sample triplicate. Average counts for each probe are then divided by the corresponding count for the reference sample (NA10851, Coriell Cell Repository) to generate a copy number estimate.

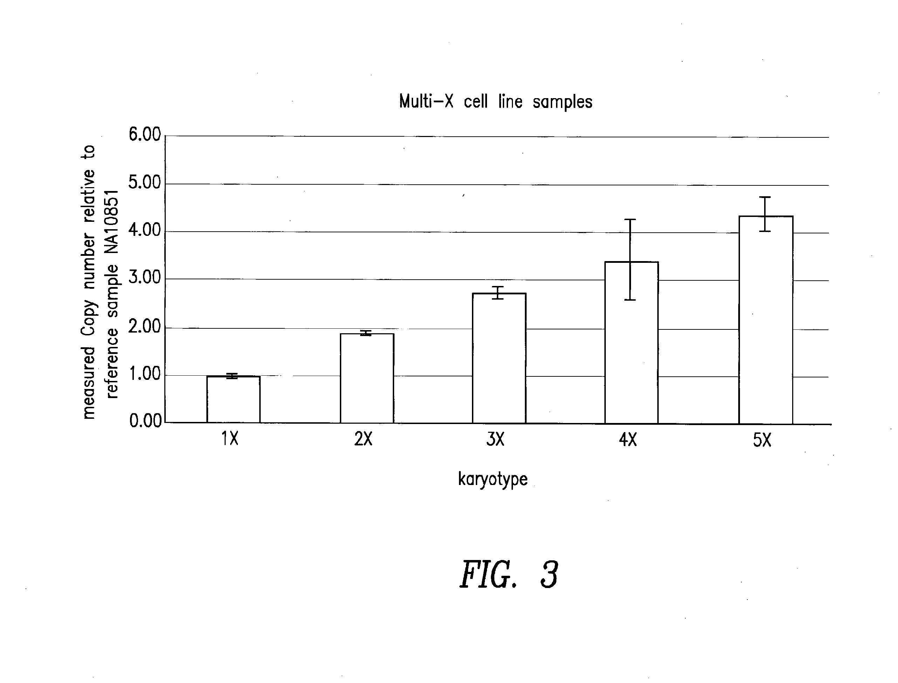

Copy number estimate is then averaged for the 3 probes to chromosome X. The graph shows the average copy number estimate and standard deviation for 3 probes to chromosome X (see Figure 3). Figures 1-3 show proof of concept that nanoreporters as described above, can be used to ascertain genomic copy number.

### Example 2: Precision Counting of Gene Copies in Normal and Cancerous Colon and Breast Tissues.

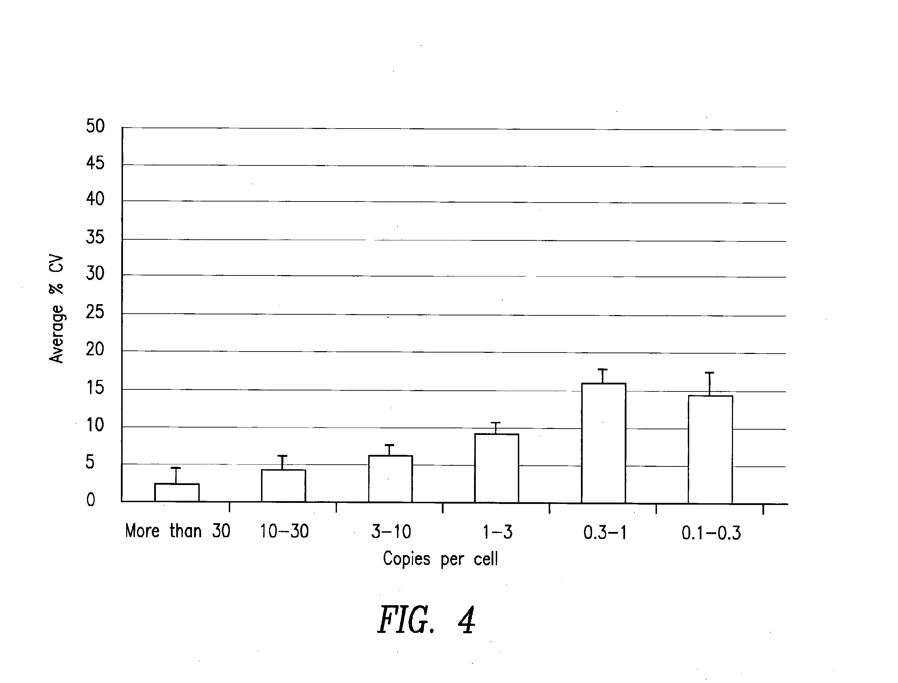

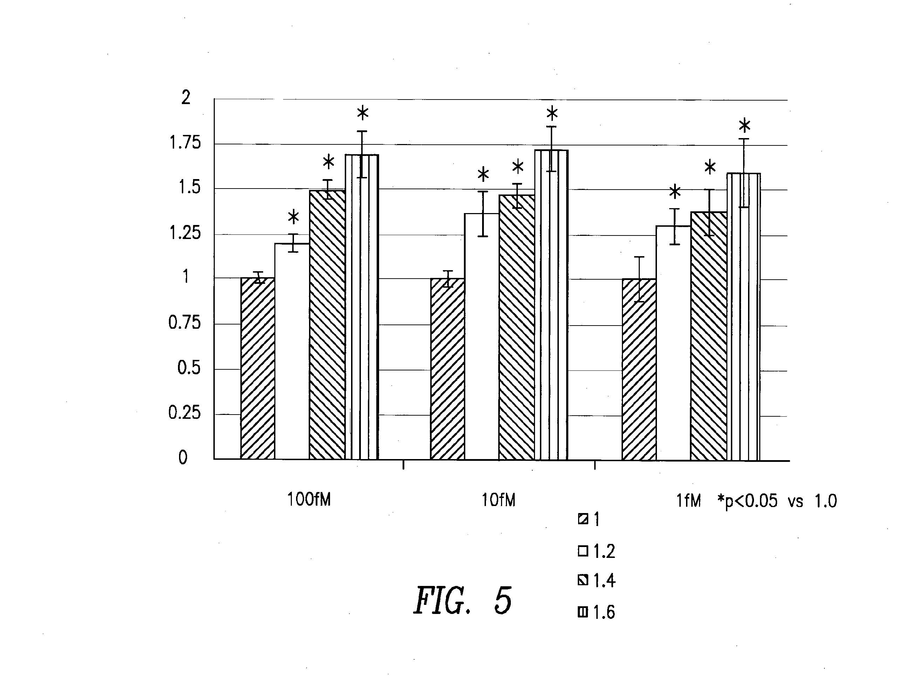

Hybridizations were performed in triplicate with normal and tumorous colon and breast tissues, with GPCR and Kinase panels (24 total hybridizations) according to the nCounter® probe pair methods described above. As shown in Figure 4, the genes were binned based on expression level, and the average %CV (coefficient of variance, standard deviation and mean) was calculated for all the genes detected in each bin. The total number of measurements (n) is indicated. Even for the 173 measurements of genes expressed at very low levels, the %CV averaged less than 15%. Likewise, small fractional fold changes can be detected according to these methods as shown in Figure 5. This demonstrates the precision of the nCounter® system in the counting of gene copies.

### Example 3: Precision Counting of Autosomal Mutations.

78 different autosomal loci, consisting of 25 HapMapped CNV regions and other regions of interest, together with 23 invariant regions (one per chromosome), were analyzed with a single CodeSet according to the nCounter® method described above. It is easy to identify the loci that vary in copy number.

Samples were, from top to bottom, NA07019, NA12877, NA10487. All the HapMap overlapping regions are 2X in these individuals. NA12877 was male; notice the single X dose in region with index 102.

### Example 4: Probe Pair Selection.

### Sequence Preparation:

A list of genomic regions was obtained. The list could be produced from many sources. For example, the list came, in some instances, from a commercial source and in others from scrutiny of genomic regions known to be involved in a particular disease of interest through a scientific literature search. The list of regions contained specific data concerning the publically available human reference genome that was used to identify the region, including the build identifier, chromosome number, start, and end coordinates.

From the list of regions, a custom track file was generated to be used for creation of a custom user track that can be viewed, for example, within the UCSC genome browser over the Internet. After loading the track file into the genome browser, unmasked raw DNA sequences were downloaded using the genome table browser. This file was generally downloaded as a multi-FASTA file containing all the regions sequences concatenated together, but sometimes it was downloaded as a single sequence FASTA file if only one region is collected.

The multi-FASTA file containing the specific DNA sequences from the regions is manipulated to strip extraneous characters from the header of each FASTA entry, leaving only necessary information such as region name, chromosome, start, and end positions. This can be accomplished using the custom Perl program replace.pl.

The multi-FASTA file of region sequences were then split into separate files, one file for each region using the custom Perl program splitfa.pl. Each file was named using the sequence name found in the header of the FASTA entry in the multi-FASTA file.

Each FASTA sequence file was then passed to the custom Perl program fragment.pl within a Linux bash shell script. The fragment.pl program created *in silico* restriction fragments of various sizes, depending on the location of restriction enzyme recognition sites in the sequence. The size of the resulting DNA fragments varied from about 0.1 kb to about 10 kb, with an approximate average size of 0.5 kb. Parameters set within fragment.pl allowed for the exclusion of fragments smaller or larger than the desired length.

When all of the sequence files had been processed, the resulting sequence fragments were concatenated into one larger file for loading into a database of DNA fragments by the custom Perl program milk.pl. This program connected to a local mySQL CNV database, to create the appropriate project within the database, and linked the data to the specific organism and genome build. It then proceeded to load the DNA sequence fragments into the database for the design of probes. The milk.pl program also read a file of region coordinates in order to create the correct data structure in the database, linking the restriction fragment sequences to the regions.

### Probe Design:

When all of the fragment sequences had been loaded into the database, the custom Perl program CNV_start_pipeline.pl ran multiple instances of the custom Perl program gauntlet.pl, that generated probe pairs specifically designed to work in the nanoString nCounter® system. Each specific probe consisted of approximately 35-50 nucleotides, with probe pairs being matched for Tm. Many probe pairs were created for each restriction fragment during the process, with the majority failing to meet rigorous specifications for the design of matched probe pairs. Those probes that pass the physical structure and matching criteria were loaded into the CNV database to be scored for fitness within the CNV assay.

### BLAT Scoring:

BLAT scoring was performed on each probe pair by concatenating the reporter probe sequence and the ghost probe sequence (each approximately 35-50 nucleotides in length). The combined reporter-ghost probe sequence was sent to a local licensed version of the BLAT program to determine the location(s) with respect to the publically available reference genome *(e.g.* hg18 or hg19). This was done to unambiguously map the probes against the reference genome and determine the potential for cross-hybridization to other regions of the genome. The output of the BLAT program for each probe pair was stored in the CNV database for subsequent scoring.

The BLAT score for each probe pair within a project was accomplished by running the custom Perl program CNV_BLAT_score.pl, that created a score in the arbitrary range of 0-100 based on the number of "hits" within a specified combination of "percent identity" (PID) and "alignment length" (AL), parameters set within the program, the location of the "hit" on the combined reporter-ghost probe sequence, and the expected number of "hits" for a particular region in the genome. Note that "hits" within the reporter probe portion of the combined sequence are weighted as more severe than those on the ghost probe portion due to technical considerations of detecting the labeled "spot" tag on the reporter probe.

### Probe Selection:

When BLAT scores had been determined and loaded into the CNV database, the selection of the "best" probes for each region was accomplished using the custom Perl program butter.pl. This program took into consideration the following parameters for picking the best probes for a given region: (i) the length of the restriction fragment the probe sequence is within that we know works best in the nCounter® system. This was based on a mathematical model of performance as determined by in-house experimentation, (ii) the location of the probe within the region to be analyzed, and (iii) the BLAT score as determined above in "BLAT scoring".

The custom Perl program butter.pl used a unique algorithm that was developed for fitness scoring of probe pairs. The algorithm included a process that allows for an expansion of the number of zones (bins) per region to facilitate selection of the requested number of probe pairs evenly distributed over the entire region using the probes that would work best in the nCounter® system according to our mathematical model of fitness. The location score was based on the number of probe pairs that had been requested per region when they submitted them for the assay design. For example, if the desired number of probes to a region was 1, then the best probe pair would be the one that (i) resides on the optimal size restriction fragment (high fragment score), (ii) is directly in the center (equal spacing from either end) of the defined region (high spacing score), and (iii) has the expected number of "hits" (high BLAT score).

The following is the flow of our unique process of probe selection for the custom CNV assay:
The user specified the value of the number of probes requested per region (N) and the maximum value of zones to try for an iterative processing of each region (MAX). Data was gathered from the database and arranged into data structures amenable to the process. For each region the following steps were performed.

For each iteration from N to MAX, a scoring matrix was created the exact length (L) of the region (in nucleotides). Then, using a natural mathematical function (absolute value of a sine wave) with the cycles specified relative to N, a value ranging from 0 to 1 was assigned to every position of the matrix. For example, if the length of the region was 1kb (1000 nucleotides) and the number of desired probes was 1, then L=1000 and N=1, then the value in the matrix position 1000/2 = 500 is equal to 1.0 (the upper limit of the absolute value of a sine function). Therefore, the best position score possible for a probe pair would be at position 500. If a probe pair were located at position 500 with all other parameters being equal amongst the other probe pairs available for the zone (ideal fragment length and ideal BLAT score), the 1 best probe pair selected would be the one located right in the center of the region, which is what we want.

For each zone (N), the following steps were performed. For each probe pair, a fitness score was calculated using the value of the fragment length model, the value of the sine wave at the position of the probe pair, and the BLAT score. As the algorithm passed through the entire region length of sequence from end to end, each probe pair score was tested against the highest score seen so far in the zone (there are N zones). If the score for the probe pair at that position was higher than the one saved previously, the probe pair was replaced with the higher scoring one. When the algorithm reached the end of each zone, the highest scoring probe pair is added to the list of those to be included in the assay. Then the next probe pair was scored.

For the next zone, when the algorithm reached the entire length of the region, the program tests if the number of probes requested (N) has been satisfied, and IF NOT, the value of N is changed to the next incremental value ofN by adding S, and the process begins all over again. The cycles continue until the maximum value of zones (MAX) has been reached. In the next iteration (N =N+S unless N=MAX). The value (N) is increased by (S) at the end of every iteration, until MAX is reached. This was then perfomed for each successive region.

### Example 5. Gene Copy Number Assay Protocol.

### AluI Restriction Digestion

A restriction enzyme fragmentation was set up. 200-600 ng of DNA in in 7 µL of sample was placed a 0.2 mL-0.5 mL PCR tube. Genomic DNA was generally at a concentration of at least 29 ng/µL prior to addition to the fragmentation reaction.

7 µL containing DNA 600 ng was dissolved in RNase free water, Tris pH 8.0 or similar. To this was added 1 µL 10X AluI Fragmentation Buffer; 1 µL 10X CNV DNA Prep Control; 1 µL AluI Fragmentation Enzyme; up to 10 µL of total volume.

The digestion was incubated at at 37° C for at least 2 hours in a heat block or thermalcycler with heated lid turned on. Samples generally were denatured upon completion of restriction digestion.

The final hybridization reaction contained the following components: 10 µL nanoreporters, 10 µL hybridization buffer, a total volume of 5 µL of sample DNA, and 5 µL Capture ProbeSet.

### Hybridization Reaction

Aliquots of both reporter probes and capture probes were removed from the freezer and thawed on benchtop at room temperature. The tubes were inverted or flicked several times to mix well and spin down reagent. A master mix was created containing 130 µL of the reporter probes and 130 µL of hybridization buffer. Hybridization buffer was added directly to nanoreporter tube. Capture probes were not added to the master mix. The master mix was inverted to mix and spun down.

12 tubes were labeled. 20 µL of mastermix was added to each of the 12 tubes.

### Thermocycler

AluI digestion tubes were denatured at 95°C for 5 minutes. The thermocycler was pre-heated to 65°C with heated lid turned on and "forever" time setting. The AluI digestion tubes were then immediately placed on ice for 2 minutes to minimize DNA renaturation and then briefly spun down.

5 µL AluI-digested DNA sample was addec to the hybridization tube. The remainder of the AluI-digested sample was generally stored at -20° C for future use.

5 µL of capture probe was added to each tube immediately before placing at 65°C. Tubes were capped and mixed the reagents by flicking with your finger to ensure complete mixing. Tubes were briefly spin down at < 1000 g and immediately placed in the 65°C thermocycler. The hybridization assays were incubated for at least 16 hours and were left at 65°C until ready for processing. Maximum hybridization time did not exceed 30 hours.

### Example 6. Optimal Size Selection of Restriction Fragments.

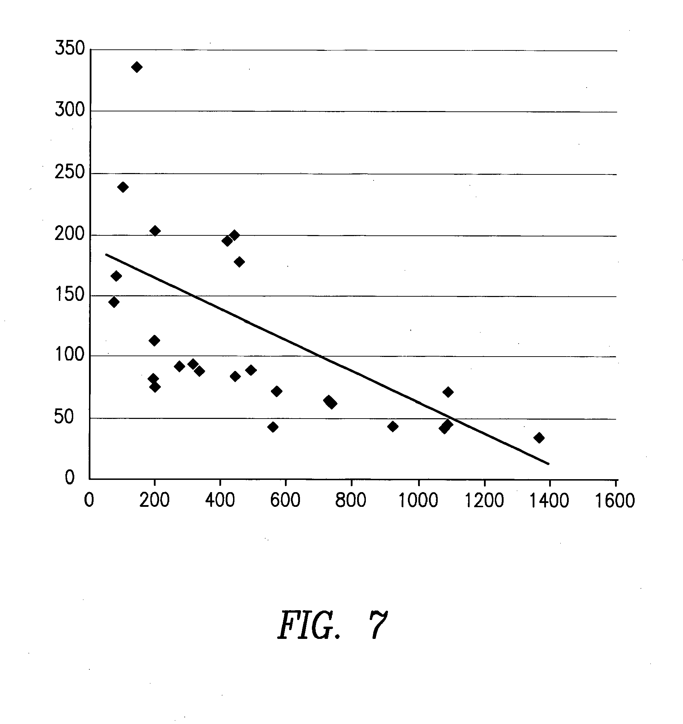

During the probe design process, target regions were analyzed for Alu1 sites (based on the human reference genome sequence) and the sizes of Alu1 fragments were predicted. One nCounter® CNV probe was designed per Alul fragment. Figure 7 shows the relationship between Alul fragment length in base pairs (x-axis) and the counts obtained via hybridization in the nCounter® system (y-axis). Based on this data, the probe design algorithm selected the optimal size fragments per genomic region.

### Example 7. Determination of Copy Number Variations (CNV using nCounter® copy number assay.

The nCounter® copy number assay was used to measure the copy number of 20 genomic regions in 50 human genomic DNA samples containing known copy number variations (CNVs). Fifty human genomic DNAs were purchased from Coriell Cell Repository. The presence of CNVs in these samples was previously determined by genome-wide analysis using microarray methodology and the data is publically available at the public website Database of Genomic Variants (http://projects.tcag.ca/variation/).

Six hundred nanograms of genomic DNA were processed via the nCounter® Copy number assay manual. Briefly, 600ng of DNA was digested with restriction enzyme Alul1, sample was denatured at 95° C for 5 minutes and added directly to an nCounter® hybridization reaction. The nCounter® CodeSet used in this experiment contained 60 probes complementary to 20 genomic regions that were selected based on known variations across these samples as well as control probes. Hybridization occurred in solution for 16 hours at 65° C. Hybridized samples were purified and imaged using nCounter® PrepStation and Digital Analyzer.

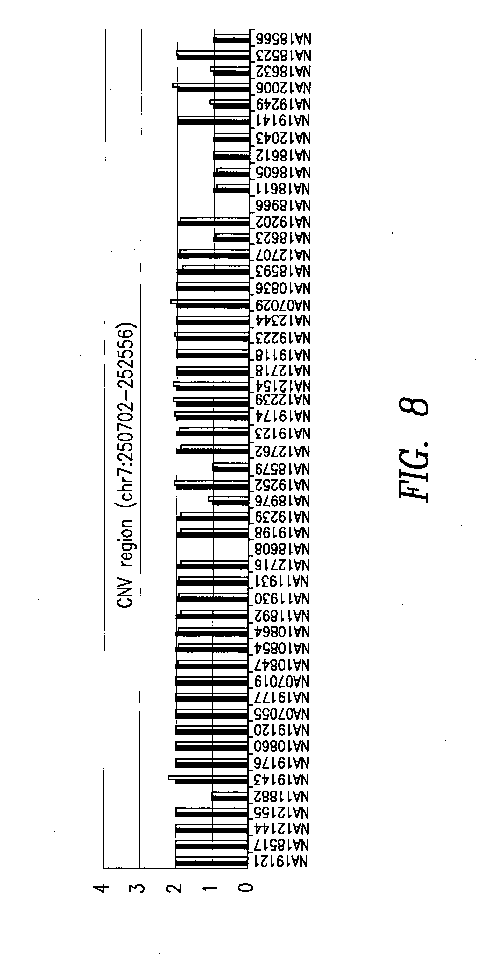

Raw data was normalized using the average signal for 10 invariant control probes. This step removes slight variations in DNA input amounts. Copy number calls were calculated by dividing the normalized counts of the test samples by the normalized counts in the reference and multiplying by 2 for autosomal chromosomes (1-22). The reference sample was NA10851 purchased from Coriell. The graph in Figure 8 shows a comparison of the copy number value (Y-axis) for a region of chromosome 7 between the nCounter® assay (light gray bars) and the public data (dark gray bars, determined by microarray). The nCounter® copy number calls were determined by averaging the copy number calls for the 3 probes designed to this region. There was 100% concordance in copy number calls in this region, across the 50 samples. Several examples of samples with heterozygous (1 copy) and homozygous deletions (zero copies) in this region are shown.

### Example 8. Karotype Panel.

The Human Karyotype Panel used below consists of 338 probe pairs designed to target known invariant regions for molecular karyotyping of the human genome. The panel has 8 probe pairs distributed across each arm of the 22 autosomes, excluding the p-arms of acrocentric chromosomes, for a total of 313 autosomal probe pairs. In addition, there are 16 X-specific and 9 Y-specific probe pairs that may be used for gender determination. The Human Karyotype Panel is provided with 10 invariant control probe pairs designed to well-characterized invariant regions on separate chromosomes to be used for normalization of digital count data, eliminating sample-to-sample variation.

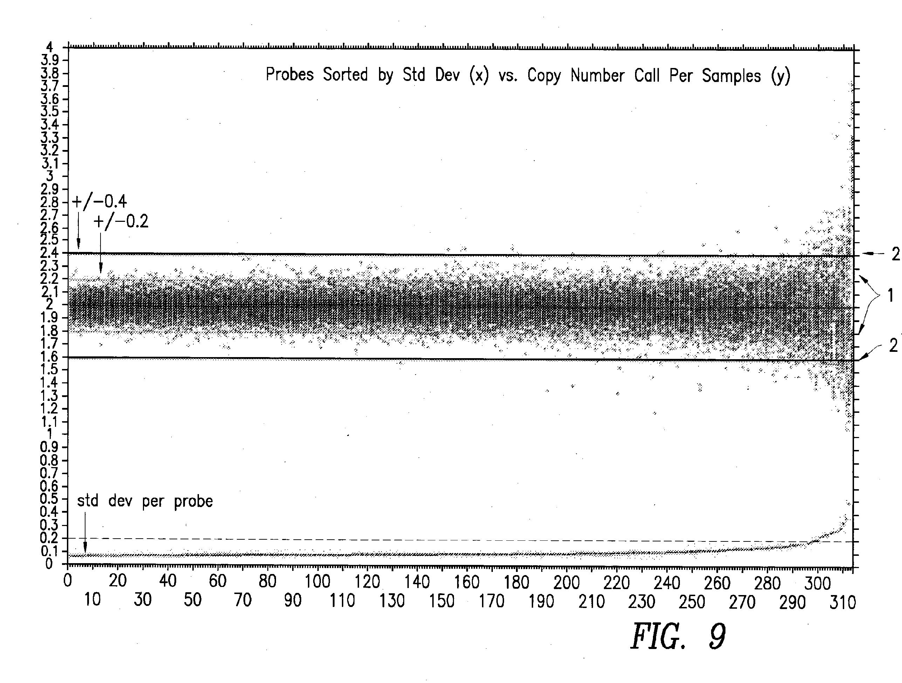

Figure 9 shows copy number calls (y axis) for all 313 autosomal probe pairs (x axis) within the Human Karyotype Panel. One hundred and two (102) "normal" human HapMap DNA samples purchased from the Coriell Institute for Medical Research were tested using the standard CNV assay protocol, described above. Normalized digital counts were compared to the male HapMap sample NA10851 to determine relative copy number calls. For each HapMap sample, the copy number call value is shown as a black cross. Lines marked as "1" represent a z-score of 1 (P<0.32) and lines marked as "2" represent a z-score of 2 (P<0.05) assuming a Gaussian distribution about the expected copy number of 2 and standard deviation of 0.2. The standard deviation of copy number calls for each probe pair is shown at the bottom of the graph, overlaying the y axis for both copy number calls and standard deviation. Probe pairs are sorted by ascending standard deviation. A cut-off value of 0.2 for standard deviation is demarcated by a gray dotted line.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims. The patent and scientific literature referred to herein establishes the knowledge that is available to those with skill in the art.

While this invention has been particularly shown and described with references to preferred embodiments thereof, it will be understood by those skilled in the art that various changes in form and details may be made therein without departing from the scope of the invention encompassed by the appended claims.

## Claims

1. A method of determining the copy number of a DNA sequence in a genome comprising:
(a) providing a first sample containing genomic DNA which is preferably mammalian genomic DNA, more preferably human genomic DNA;
(b) fragmenting the genomic DNA;
(c) denaturing the genomic DNA;
(d) providing a reference sample comprising fragmented genomic DNA wherein the copy number of the fragment of genomic DNA to be detected is known;
(e) providing a first nanoreporter comprising a first probe and a second probe,
wherein said first probe comprises
(i) a first label attachment region to which are attached one or more label monomers that emit light constituting a first signal;
(ii) a second label attachment region, which is non-over-lapping with the first label attachment region, to which are attached one or more label monomers that emit light constituting a second signal; and
(iii) a first target-specific sequence attached to the first probe, wherein the first target-specific sequence specifically hybridises to the fragmented genomic DNA sequence to be detected;
and wherein said second probe comprises
(i) a second target-specific sequence, wherein the second target-specific sequence specifically hybridises to the fragmented genomic DNA sequence to be detected; and
(ii) a first affinity tag;
wherein the first target specific sequence of the first probe and the second target-specific sequence of the second probe hybridize to different regions of the same fragmented genomic DNA sequence to be detected;
(f) contacting the first probe and the second probe with the fragmented genomic DNA of the first sample and the reference sample wherein the contact is made under conditions sufficient for hybridisation of the first target specific sequence and the second target specific sequence to a fragment of the fragmented genomic DNA comprising the genomic DNA sequence to be detected to form a first hybridized complex, wherein said contacting is preferably performed in solution;
(g) purifying the first hybridized complex through the first affinity tag, thereby separating the first hybridized complex from unbound materials;
(h) stretching the first hybridized complex using a flow-stretch, receding meniscus, or electro-stretch technique, thereby spatially separating said label monomers,
(i) measuring a signal from the first probe, wherein said signal uniquely identifies the at least one fragment of the fragmented genomic DNA comprising the genomic DNA sequence to be detected, and
(j) comparing the signal from the first sample to the signal from the reference sample, wherein the copy number of the first sample is determined by correlating the signal from the first sample with the signal from the reference sample.

2. The method of claim 1, wherein the genomic DNA sample is unamplified.

3. The method of claim 1, wherein the fragmentation is performed by restriction enzyme digestion, wherein the restriction enzyme is optionally Alu1 or Bfal, or wherein the fragmentation is performed chemically, by mechanical shearing or sonication.

4. The method of claim 1, wherein the reference sample is a synthetic nucleic acid sample or a biological genomic DNA sample.

5. A method according to claim 1 further comprising normalising the signal generated by the steps:
(a) providing at least a second nanoreporter comprising a third probe and a fourth probe, wherein said third probe comprises
(i) a third label attachment region to which are attached one or more label monomers that emit light constituting a third signal;
(ii) a fourth label attachment region, which is non-over-lapping with the third label attachment region, to which are attached one or more label monomers that emit light constituting a fourth signal; and
(iii) a third target-specific sequence attached to the third probe, wherein the third target-specific sequence specifically hybridises to a first DNA fragment from a copy number invariant region of the genome;
and wherein said fourth probe comprises
(i) a fourth target-specific sequence, wherein the fourth target-specific sequence specifically hybridises to the first DNA fragment from a copy number invariant region of the genome; and
(iv) a second affinity tag
wherein the third target specific sequence of the third probe and the fourth target-specific sequence of the fourth probe hybridize to different regions of the same fragment from a copy number invariant region of the genome;
(b) contacting the third probe and the fourth probe with the fragmented genomic DNA from the first sample and the reference sample wherein the contact is made under conditions sufficient for hybridisation of the third target specific sequence and the fourth target specific sequence to the first DNA fragment from a copy number invariant region of the genome to form a second hybridized complex;
(c) purifying the second hybridized complex through the second affinity tag, thereby separating the second hybridized complex from unbound materials;
(d) stretching the third probe hybridised to the first DNA fragment from a copy number invariant region of the genome using a flow-stretch, receding meniscus, or electro-stretch technique, thereby spatially separating said label monomers;
(e) measuring a signal from the third probe, wherein said signal uniquely identifies the first DNA fragment from a copy number invariant region of the genome; and
(f) comparing the signal from the second nanoreporter contacted with the first sample and the second nanoreporter contacted with the reference sample, wherein the number of multiples of the quantity of signal from the second nanoreporter contacted with the first sample compared to the quantity of signal from the second nanoreporter contacted with the reference sample normalises the signal from the first nanoreporter contacted with the first sample.

6. The method of claim 5, wherein the first DNA fragment from a copy number invariant region of the genome comprises a nucleic acid sequence selected from the group consisting of SEQ ID NOs: 1-66, or a nucleic acid sequence selected from the group consisting of SEQ ID NOs: 2, 5, 7, 12, 13, 17, 19, 24, 25, 28, 32, 36, 38, 40, 44, 46, 50, 52, 56, 58, 62 and 66 or a nucleic acid sequence selected from the group consisting of SEQ ID NOs: 2, 5, 13, 19, 28, 46, 50, 56, 58 and 66.

7. The method of claim 1, wherein the signal generated from the first nanoreporter hybridised to the at least one fragment of the fragmented genomic DNA comprising the genomic DNA sequence to be detected comprises a mixture of two or more different label monomers.

8. The method of claim 5, wherein the signal generated from the second nanoreporter hybridised to the first DNA fragment from a copy number invariant region of the genome comprises a mixture of two or more different label monomers.

9. The method of claim 1 or 5, wherein said labels are fluorescent.

10. A method according to claim 1 for detecting two or more DNA sequences in a genome further comprising:
(a) providing two or more nanoreporters that each specifically hybridise to a distinct genomic DNA sequence to be detected;
(b) measuring a signal from the two or more nanoreporters, wherein said signal uniquely identifies each of the corresponding distinct genomic DNA sequences thereby detecting two or more DNA sequences in a genome; and
(c) determining the copy number by comparing the signal from the first sample to the signal from the reference sample.

11. A method according to claim 5 for normalising the signal generated in claim 10 further comprising comparing the signal from the second nanoreporter contacted with the first sample and the second nanoreporter contacted with the reference sample, wherein the number of multiples of the quantity of signal from the second nanoreporter contacted with the first sample compared to the quantity of signal from the second nanoreporter contacted with the reference sample normalises the signal from the first nanoreporter contacted with the first sample.

12. The method of claim 11, wherein the first DNA fragment from a copy number invariant region of the genome comprises a nucleic acid sequence selected from the group consisting of SEQ ID NOs: 1-66.

13. A kit, comprising
(a) a first nanoreporter comprising a first probe and a second probe, wherein said first probe comprises
(i) a first label attachment region to which are attached one or more label monomers that emit light constituting a first signal;
(ii) a second label attachment region, which is non-over-lapping with the first label attachment region, to which are attached one or more label monomers that emit light constituting a second signal; and
(iii) a first target-specific sequence attached to the first probe, wherein the first target-specific sequence specifically hybridises to a target DNA sequence;
and wherein said second probe comprises
(i) a second target-specific sequence, wherein the first target-specific sequence specifically hybridises to the target DNA sequence; and
(ii) a first affinity tag;
wherein the first target specific sequence of the first probe and the second target specific sequence of the second probe hybridize to different regions of the same target DNA;
(b) a second nanoreporter comprising a third probe and a fourth probe, wherein said third probe comprises
(i) a third label attachment region to which are attached one or more label monomers that emit light constituting a third signal;
(ii) a fourth label attachment region, which is non-over-lapping with the third label attachment region, to which are attached one or more label monomers that emit light constituting a fourth signal; and
(iii) a third target-specific sequence attached to the third probe, wherein the third target-specific sequence specifically hybridises to a first DNA fragment from a copy number invariant region of the genome;
and wherein said fourth probe comprises
(i) a fourth target-specific sequence, wherein the fourth target-specific sequence specifically hybridises to the first DNA fragment from a copy number invariant region of the genome; and
(iv) a second affinity tag;
wherein the third target specific sequence of the third probe and the fourth target-specific sequence of the fourth probe hybridize to different regions of the same fragment from a copy number invariant region of the genome; and
(c) a restriction enzyme.

14. A kit according to claim 13 wherein said first DNA fragment from a copy number invariant region of the genome to which the third or fourth target sequence specifically hybridizes comprises an isolated nucleic acid molecule comprising at least 50 nucleotides of a sequence selected from the group consisting of SEQ ID NO: 1-66.

15. The method of any of claims 1 to 4 further comprising immobilising and purifying the first probe hybridised to the fragment to be detected using an affinity reagent which binds to the affinity tag, thereby separating the first probe hybridised to the fragment to be detected from unbound probe or fragmented DNA.

16. The method of any of claims 1 to 12 wherein the fragmented genomic DNA of the biological sample and of the reference sample are both on average between 100 and 500 pairs.

## Patentansprüche

1. Verfahren zum Bestimmen der Kopienzahl einer DNA-Sequenz in einem Genom, umfassend:
(a) Bereitstellen einer ersten Probe, die genomische DNA enthält, die bevorzugt genomische DNA eines Säugers, besonders bevorzugt humane genomische DNA ist;
(b) Fragmentieren der genomischen DNA;
(c) Denaturieren der genomischen DNA;
(d) Bereitstellen einer Referenzprobe, die fragmentierte genomische DNA umfasst, wobei die Kopienzahl des nachzuweisenden Fragments genomischer DNA bekannt ist;
(e) Bereitstellen eines ersten Nanoreporters, der eine erste Sonde und eine zweite Sonde umfasst, wobei die erste Sonde umfasst:
(i) eine erste Markerbindungsregion, an die ein oder mehrere Markermonomere gebunden sind, die Licht emittieren, das ein erstes Signal darstellt;
(ii) eine zweite Markerbindungsregion, die nicht mit der ersten Markerbindungsregion überlappt, an die ein oder mehrere Markermonomere gebunden sind, die Licht emittieren, das ein zweites Signal darstellt; und
(iii) eine erste targetspezifische Sequenz, die an die erste Sonde gebunden ist, wobei die erste targetspezifische Sequenz spezifisch an die nachzuweisende fragmentierte genomische DNA-Sequenz hybridisiert;
und wobei die zweite Sonde umfasst:
(i) eine zweite targetspezifische Sequenz, wobei die zweite targetspezifische Sequenz spezifisch an die nachzuweisende fragmentierte genomische DNA-Sequenz hybridisiert; und
(ii) ein erstes Affinitäts-Tag; wobei die erste targetspezifische Sequenz der ersten Sonde und die zweite targetspezifische Sequenz der zweiten Sonde an unterschiedliche Regionen der gleichen nachzuweisenden fragmentierten genomischen DNA-Sequenz hybridisieren;
(f) Kontaktieren der ersten Sonde und der zweiten Sonde mit der fragmentierten genomischen DNA der ersten Probe und der Referenzprobe, wobei der Kontakt unter Bedingungen erfolgt, die zur Hybridisierung der ersten targetspezifischen Sequenz und der zweiten targetspezifischen Sequenz an ein Fragment der fragmentierten genomischen DNA, das die nachzuweisende genomische DNA-Sequenz umfasst, genügen, um einen ersten hybridisierten Komplex zu bilden, wobei das Kontaktieren bevorzugt in Lösung erfolgt;
(g) Reinigen des ersten hybridisierten Komplexes durch das erste Affinitäts-Tag, wodurch der erste hybridisierte Komplex von ungebundenem Material abgetrennt wird;
(h) Strecken des ersten hybridisierten Komplexes mit einem "Flow-Stretch"-Verfahren, "Receding-Meniscus"-Verfahren oder "Elektro-Stretch"-Verfahren, wodurch die Markermonomere räumlich getrennt werden;
(i) Messen eines Signals von der ersten Sonde, wobei das Signal eindeutig das wenigstens eine Fragment der fragmentierten genomischen DNA identifiziert, das die nachzuweisende genomische DNA-Sequenz umfasst; und
(j) Vergleichen des Signals von der ersten Probe mit dem Signal von der Referenzprobe, wobei die Kopienzahl der ersten Probe durch Korrelieren des Signals von der ersten Probe mit dem Signal von der Referenzprobe bestimmt wird.

2. Verfahren nach Anspruch 1, wobei die genomische DNA-Probe nicht amplifiziert ist.

3. Verfahren nach Anspruch 1, wobei die Fragmentierung durch Restriktionsenzymverdau erfolgt, wobei das Restriktionsenzym optional Alu I oder Bfa I ist, oder wobei die Fragmentierung chemisch, durch mechanisches Scheren oder durch Ultraschall erfolgt.

4. Verfahren nach Anspruch 1, wobei die Referenzprobe eine synthetische Nukleinsäureprobe oder eine biologische genomische DNA-Probe ist.

5. Verfahren nach Anspruch 1, weiterhin umfassend Normieren des generierten Signals durch die Schritte:
(a) Bereitstellen wenigstens eines zweiten Nanoreporters, der eine dritte Sonde und eine vierte Sonde umfasst, wobei die dritte Sonde umfasst:
(i) eine dritte Markerbindungsregion, an die ein oder mehrere Markermonomere gebunden sind, die Licht emittieren, das ein drittes Signal darstellt;
(ii) eine vierte Markerbindungsregion, die nicht mit der dritten Markerbindungsregion überlappt, an die ein oder mehrere Markermonomere gebunden sind, die Licht emittieren, das ein viertes Signal darstellt; und
(iii) eine dritte targetspezifische Sequenz, die an die dritte Sonde gebunden ist, wobei die dritte targetspezifische Sequenz spezifisch an ein erstes DNA-Fragment von einer in der Kopienzahl invarianten Region des Genoms hybridisiert;
und wobei die vierte Sonde umfasst:
(i) eine vierte targetspezifische Sequenz, wobei die vierte targetspezifische Sequenz spezifisch an das erste DNA-Fragment von einer in der Kopienzahl invarianten Region des Genoms hybridisiert; und
(iv) ein zweites Affinitäts-Tag;
wobei die dritte targetspezifische Sequenz der dritten Sonde und die vierte targetspezifische Sequenz der vierten Sonde an unterschiedliche Regionen des gleichen Fragments von einer in der Kopienzahl invarianten Region des Genoms hybridisieren;
(b) Kontaktieren der dritten Sonde und der vierten Sonde mit der fragmentierten genomischen DNA von der ersten Probe und der Referenzprobe, wobei der Kontakt unter Bedingungen erfolgt, die zur Hybridisierung der dritten targetspezifischen Sequenz und der vierten targetspezifischen Sequenz an das erste DNA-Fragment von einer in der Kopienzahl invarianten Region des Genoms genügen, um einen zweiten hybridisierten Komplex zu bilden;
(c) Reinigen des zweiten hybridisierten Komplexes durch das zweite Affinitäts-Tag, wodurch der zweite hybridisierte Komplex von ungebundenem Material abgetrennt wird;
(d) Strecken der dritten Sonde, die an das erste DNA-Fragment von einer in der Kopienzahl invarianten Region des Genoms gebunden ist, mit einem "Flow-Stretch"-Verfahren, "Receding-Meniscus"-Verfahren oder "Elektro-Stretch"-Verfahren, wodurch die Markermonomere räumlich getrennt werden;
(e) Messen eines Signals von der dritten Sonde, wobei das Signal eindeutig das erste DNA-Fragment von einer in der Kopienzahl invarianten Region des Genoms identifiziert; und
(f) Vergleichen des Signals von dem zweiten Nanoreporter, der mit der ersten Probe kontaktiert ist, und dem zweiten Nanoreporter, der mit der Referenzprobe kontaktiert ist, wobei die Anzahl an Vielfachen der Signalstärke von dem zweiten Nanoreporter, der mit der ersten Probe kontaktiert ist, im Vergleich mit der Signalstärke von dem zweiten Nanoreporter, der mit der Referenzprobe kontaktiert ist, das Signal von dem ersten Nanoreporter, der mit der ersten Probe kontaktiert ist, normiert.

6. Verfahren nach Anspruch 5, wobei das erste DNA-Fragment von einer in der Kopienzahl invarianten Region des Genoms eine Nukleinsäuresequenz umfasst, die ausgewählt ist aus der Gruppe bestehend aus SEQ ID NO: 1-66, oder eine Nukleinsäuresequenz, die ausgewählt ist aus der Gruppe bestehend aus SEQ ID NO: 2, 5, 7, 12, 13, 17, 19, 24, 25, 28, 32, 36, 38, 40, 44, 46, 50, 52, 56, 58, 62 und 66, oder eine Nukleinsäuresequenz, die ausgewählt ist aus der Gruppe bestehend aus SEQ ID NO: 2, 5, 13, 19, 28, 46, 50, 56, 58 und 66.

7. Verfahren nach Anspruch 1, wobei das Signal, das von dem ersten Nanoreporter generiert wird, der an das wenigstens eine Fragment der fragmentierten genomischen DNA hybridisiert ist, das die nachzuweisende genomische DNA-Sequenz umfasst, eine Mischung aus zwei oder mehr unterschiedlichen Markermonomeren umfasst.

8. Verfahren nach Anspruch 5, wobei das Signal, das von dem zweiten Nanoreporter generiert wird, der an das erste DNA-Fragment von einer in der Kopienzahl invarianten Region des Genoms hybridisiert ist, eine Mischung aus zwei oder mehr unterschiedlichen Markermonomeren umfasst.

9. Verfahren nach Anspruch 1 oder 5, wobei die Marker fluoreszierend sind.

10. Verfahren nach Anspruch 1 zum Nachweis von zwei oder mehr DNA-Sequenzen in einem Genom, weiterhin umfassend:
(a) Bereitstellen von zwei oder mehr Nanoreportern, die jeweils spezifisch an eine distinkte nachzuweisende genomische DNA-Sequenz binden;
(b) Messen eines Signals von den zwei oder mehr Nanoreportern, wobei das Signal eindeutig jede der entsprechenden distinkten genomischen DNA-Sequenzen identifiziert, wodurch zwei oder mehr DNA-Sequenzen in einem Genom nachgewiesen werden; und
(c) Bestimmen der Kopienzahl durch Vergleichen des Signals von der ersten Probe mit dem Signal von der Referenzprobe.

11. Verfahren nach Anspruch 5 zum Normieren des in Anspruch 10 generierten Signals, weiterhin umfassend Vergleichen des Signals von dem zweiten Nanoreporter, der mit der ersten Probe kontaktiert ist, und dem zweiten Nanoreporter, der mit der Referenzprobe kontaktiert ist, wobei die Anzahl an Vielfachen der Signalstärke von dem zweiten Nanoreporter, der mit der ersten Probe kontaktiert ist, im Vergleich mit der Signalstärke von dem zweiten Nanoreporter, der mit der Referenzprobe kontaktiert ist, das Signal von dem ersten Nanoreporter, der mit der ersten Probe kontaktiert ist, normiert.

12. Verfahren nach Anspruch 11, wobei das erste DNA-Fragment von einer in der Kopienzahl invarianten Region des Genoms eine Nukleinsäuresequenz umfasst, die ausgewählt ist aus der Gruppe bestehend aus SEQ ID NO: 1-66.

13. Kit, umfassend:
(a) einen ersten Nanoreporter, der eine erste Sonde und eine zweite Sonde umfasst, wobei die erste Sonde umfasst:
(i) eine erste Markerbindungsregion, an die ein oder mehrere Markermonomere gebunden sind, die Licht emittieren, das ein erstes Signal darstellt;
(ii) eine zweite Markerbindungsregion, die nicht mit der ersten Markerbindungsregion überlappt, an die ein oder mehrere Markermonomere gebunden sind, die Licht emittieren, das ein zweites Signal darstellt; und
(iii) eine erste targetspezifische Sequenz, die an die erste Sonde gebunden ist, wobei die erste targetspezifische Sequenz spezifisch an eine Target-DNA-Sequenz hybridisiert;
und wobei die zweite Sonde umfasst:
(i) eine zweite targetspezifische Sequenz, wobei die erste targetspezifische Sequenz spezifisch an die Target-DNA-Sequenz hybridisiert; und
(ii) ein erstes Affinitäts-Tag;
wobei die erste targetspezifische Sequenz der ersten Sonde und die zweite targetspezifische Sequenz der zweiten Sonde an unterschiedliche Regionen der gleichen Target-DNA hybridisieren;
(b) einen zweiten Nanoreporter, der eine dritte Sonde und eine vierte Sonde umfasst, wobei die dritte Sonde umfasst:
(i) eine dritte Markerbindungsregion, an die ein oder mehrere Markermonomere gebunden sind, die Licht emittieren, das ein drittes Signal darstellt;
(ii) eine vierte Markerbindungsregion, die nicht mit der dritten Markerbindungsregion überlappt, an die ein oder mehrere Markermonomere gebunden sind, die Licht emittieren, das ein viertes Signal darstellt; und
(iii) eine dritte targetspezifische Sequenz, die an die dritte Sonde gebunden ist, wobei die dritte targetspezifische Sequenz spezifisch an ein erstes DNA-Fragment von einer in der Kopienzahl invarianten Region des Genoms hybridisiert;
und wobei die vierte Sonde umfasst:
(i) eine vierte targetspezifische Sequenz, wobei die vierte targetspezifische Sequenz spezifisch an das erste Fragment von einer in der Kopienzahl invarianten Region des Genoms hybridisiert; und
(iv) ein zweites Affinitäts-Tag;
wobei die dritte targetspezifische Sequenz der dritten Sonde und die vierte targetspezifische Sequenz der vierten Sonde an unterschiedliche Regionen des gleichen Fragments von einer in der Kopienzahl invarianten Region des Genoms hybridisieren; und
(c) ein Restriktionsenzym.

14. Kit nach Anspruch 13, wobei das erste DNA-Fragment von einer in der Kopienzahl invarianten Region des Genoms, an das die dritte oder vierte targetspezifische Sequenz spezifisch hybridisiert, ein isoliertes Nukleinsäuremolekül umfasst, das wenigstens 50 Nukleotide einer Sequenz umfasst, die ausgewählt ist aus der Gruppe bestehend aus SEQ ID NO: 1-66.

15. Verfahren nach einem der Ansprüche 1 bis 4, weiterhin umfassend Immobilisieren und Reinigen der ersten Sonde, die an das nachzuweisende Fragment hybridisiert ist, unter Verwendung eines Affinitätsreagens, das an das Affinitäts-Tag bindet, wodurch die erste Sonde, die an das nachzuweisende Fragment hybridisiert ist, von ungebundener Sonde oder fragmentierter DNA abgetrennt wird.

16. Verfahren nach einem der Ansprüche 1 bis 12, wobei die fragmentierte genomische DNA der biologischen Probe und der Referenzprobe beide durchschnittlich zwischen 100 und 500 Paare haben.

## Revendications

1. Procédé pour déterminer le nombre de copies d'une séquence d'ADN dans un génome, comprenant :
(a) la fourniture d'un premier échantillon contenant de l'ADN génomique qui est de préférence de l'ADN génomique de mammifère, plus préférablement de l'ADN génomique humain ;
(b) la fragmentation de l'ADN génomique ;
(c) la dénaturation de l'ADN génomique ;
(d) la fourniture d'un échantillon de référence comprenant de l'ADN génomique fragmenté, le nombre de copies du fragment d'ADN génomique devant être détecté étant connu ;
(e) la fourniture d'un premier nanorapporteur comprenant une première sonde et une deuxième sonde,
la première sonde comprenant
(i) une première région de rattachement de marqueurs à laquelle est/sont rattaché(s) un ou plusieurs monomère(s) marqueur(s) qui émet(tent) une lumière constituant un premier signal ;
(ii) une deuxième région de rattachement de marqueurs qui ne chevauche pas la première région de rattachement de marqueurs, à laquelle est/sont rattaché(s) un ou plusieurs monomère(s) marqueur(s) qui émet(tent) une lumière constituant un deuxième signal ; et
(iii)une première séquence spécifique d'une cible rattachée à la première sonde, la première séquence spécifique d'une cible s'hybridant spécifiquement à la séquence d'ADN génomique fragmenté devant être détectée ;
et ladite deuxième sonde comprenant
(i) une deuxième séquence spécifique d'une cible, la deuxième séquence spécifique d'une cible s'hybridant spécifiquement à la séquence d'ADN génomique fragmenté devant être détectée ; et
(ii) une première étiquette d'affinité ;
la première séquence spécifique d'une cible de la première sonde et la deuxième séquence spécifique d'une cible de la deuxième sonde s'hybridant à des régions différentes de la même séquence d'ADN génomique fragmenté devant être détectée ;
(f) la mise en contact de la première sonde et de la deuxième sonde avec l'ADN génomique fragmenté du premier échantillon et de l'échantillon de référence, le contact étant effectué dans des conditions suffisantes pour l'hybridation de la première séquence spécifique d'une cible et de la deuxième séquence spécifique d'une cible à un fragment de l'ADN génomique fragmenté comprenant la séquence d'ADN génomique devant être détectée de manière à former un premier complexe hybridé, ladite mise en contact étant de préférence réalisée en solution ;
(g) la purification du premier complexe hybridé par l'intermédiaire de la première étiquette d'affinité, en séparant ainsi le premier complexe hybridé d'avec les matériaux non liés ;
(h) l'étirage du premier complexe hybridé en utilisant une technique d'étirage en écoulement, une technique du ménisque descendant, ou une technique d'électro-étirage, en séparant ainsi spatialement lesdits monomères marqueurs ;
(i) la mesure d'un signal provenant de la première sonde, ledit signal identifiant de manière unique l'au moins un fragment de l'ADN génomique fragmenté comprenant la séquence d'ADN génomique devant être détectée ; et
(j) la comparaison du signal du premier échantillon au signal de l'échantillon de référence, le nombre de copies du premier échantillon étant déterminé par corrélation du signal du premier échantillon avec le signal de l'échantillon de référence.

2. Procédé selon la revendication 1, dans lequel l'échantillon d'ADN génomique n'est pas amplifié.

3. Procédé selon la revendication 1, dans lequel la fragmentation est effectuée par une digestion avec une enzyme de restriction, l'enzyme de restriction étant éventuellement Alul ou Bfa1, ou dans lequel la fragmentation est effectuée chimiquement, par cisaillement mécanique ou sonification.

4. Procédé selon la revendication 1, dans lequel l'échantillon de référence est un échantillon d'acide nucléique synthétique ou un échantillon d'ADN génomique biologique.

5. Procédé selon la revendication 1, comprenant en outre la normalisation du signal généré par les étapes :
(a) de fourniture d'au moins un deuxième nanorapporteur comprenant une troisième sonde et une quatrième sonde, ladite troisième sonde comprenant
(i) une troisième région de rattachement de marqueurs à laquelle est/sont rattaché(s) un ou plusieurs monomère(s) marqueur(s) qui émet(tent) une lumière constituant un troisième signal ;
(ii) une quatrième région de rattachement de marqueurs qui ne chevauche pas la troisième région de rattachement de marqueurs, à laquelle est/sont rattaché(s) un ou plusieurs monomère(s) marqueur(s) qui émet(tent) une lumière constituant un quatrième signal ; et
(iii)une troisième séquence spécifique d'une cible rattachée à la troisième sonde, la troisième séquence spécifique d'une cible s'hybridant spécifiquement à un premier fragment d'ADN provenant d'une région du génome dont le nombre de copies ne varie pas ;
et ladite quatrième sonde comprenant
(i) une quatrième séquence spécifique d'une cible, la quatrième séquence spécifique d'une cible s'hybridant spécifiquement au premier fragment d'ADN provenant d'une région du génome dont le nombre de copies ne varie pas ; et
(ii) une deuxième étiquette d'affinité ;
la troisième séquence spécifique d'une cible de la troisième sonde et la quatrième séquence spécifique d'une cible de la quatrième sonde s'hybridant à des régions différentes du même fragment provenant d'une région du génome dont le nombre de copies ne varie pas ;
(b) la mise en contact de la troisième sonde et de la quatrième sonde avec l'ADN génomique fragmenté du premier échantillon et de l'échantillon de référence, le contact étant effectué dans des conditions suffisantes pour l'hybridation de la troisième séquence spécifique d'une cible et de la quatrième séquence spécifique d'une cible au premier fragment d'ADN provenant d'une région du génome dont le nombre de copies ne varie pas de manière à former un deuxième complexe hybridé ;
(c) la purification du deuxième complexe hybridé par l'intermédiaire de la deuxième étiquette d'affinité, en séparant ainsi le deuxième complexe hybridé d'avec les matériaux non liés ;
(d) l'étirage de la troisième sonde hybridée au premier fragment d'ADN provenant d'une région du génome dont le nombre de copies ne varie pas en utilisant une technique d'étirage en écoulement, une technique du ménisque descendant, ou une technique d'électro-étirage, en séparant ainsi spatialement lesdits monomères marqueurs ;
(e) la mesure d'un signal provenant de la troisième sonde, ledit signal identifiant de manière unique le premier fragment d'ADN provenant d'une région du génome dont le nombre de copies ne varie pas ; et
(f) la comparaison du signal du deuxième nanorapporteur mis en contact avec le premier échantillon et du deuxième nanorapporteur mis en contact avec l'échantillon de référence, le nombre de multiples de la quantité de signal du deuxième nanorapporteur mis en contact avec le premier échantillon, en comparaison avec la quantité de signal du deuxième nanorapporteur mis en contact avec l'échantillon de référence, normalisant le signal du premier nanorapporteur mis en contact avec le premier échantillon.

6. Procédé selon la revendication 5, dans lequel le premier fragment d'ADN provenant d'une région du génome dont le nombre de copies ne varie pas comprend une séquence d'acide nucléique choisie dans l'ensemble constitué par les SEQ ID NO : 1 à 66, ou une séquence d'acide nucléique choisie dans l'ensemble constitué par les SEQ ID NO : 2, 5, 7, 12, 13, 17, 19, 24, 25, 28, 32, 36, 38, 40, 44, 46, 50, 52, 56, 58, 62 et 66, ou une séquence d'acide nucléique choisie dans l'ensemble constitué par les SEQ ID NO : 2, 5, 13, 19, 28, 46, 50, 56, 58 et 66.

7. Procédé selon la revendication 1, dans lequel le signal généré par le premier nanorapporteur hybridé à l'au moins un fragment de l'ADN génomique fragmenté comprenant la séquence d'ADN génomique devant être détectée comprend un mélange de deux ou plus de deux monomères marqueurs différents.

8. Procédé selon la revendication 5, dans lequel le signal généré par le deuxième nanorapporteur hybridé au premier fragment d'ADN provenant d'une région du génome dont le nombre de copies ne varie pas comprend un mélange de deux ou plus de deux monomères marqueurs différents.

9. Procédé selon la revendication 1 ou 5, dans lequel lesdits marqueurs sont fluorescents.

10. Procédé selon la revendication 1 pour détecter deux ou plus de deux séquences d'ADN dans un génome, comprenant en outre :
(a) la fourniture de deux ou plus de deux nanorapporteurs qui s'hybrident chacun spécifiquement à une séquence d'ADN génomique distincte devant être détectée ;
(b) la mesure d'un signal provenant des deux ou plus de deux nanorapporteurs, ledit signal identifiant de manière unique chacune des séquences d'ADN génomique distinctes correspondantes en détectant ainsi deux ou plus de deux séquences d'ADN dans un génome ; et
(c) la détermination du nombre de copies en comparant le signal du premier échantillon avec le signal de l'échantillon de référence.

11. Procédé selon la revendication 5 pour normaliser le signal généré dans la revendication 10, comprenant en outre la comparaison du signal du deuxième nanorapporteur mis en contact avec le premier échantillon et celui du deuxième nanorapporteur mis en contact avec l'échantillon de référence, dans lequel le nombre de multiples de la quantité de signal du deuxième nanorapporteur mis en contact avec le premier échantillon, en comparaison avec la quantité de signal du deuxième nanorapporteur mis en contact avec l'échantillon de référence, normalise le signal du premier nanorapporteur mis en contact avec le premier échantillon.

12. Procédé selon la revendication 11, dans lequel le premier fragment d'ADN provenant d'une région du génome dont le nombre de copies ne varie pas comprend une séquence d'acide nucléique choisie dans l'ensemble constitué par les SEQ ID NO : 1 à 66.

13. Kit comprenant
(a) un premier nanorapporteur comprenant une première sonde et une deuxième sonde,
ladite première sonde comprenant
(i) une première région de rattachement de marqueurs à laquelle est/sont rattaché(s) un ou plusieurs monomère(s) marqueur(s) qui émet(tent) une lumière constituant un premier signal ;
(ii) une deuxième région de rattachement de marqueurs qui ne chevauche pas la première région de rattachement de marqueurs, à laquelle est/sont rattaché(s) un ou plusieurs monomère(s) marqueur(s) qui émet(tent) une lumière constituant un deuxième signal ; et
(iii)une première séquence spécifique d'une cible rattachée à la première sonde, la première séquence spécifique d'une cible s'hybridant spécifiquement à une séquence d'ADN cible ;
et ladite deuxième sonde comprenant
(i) une deuxième séquence spécifique d'une cible, la première séquence spécifique d'une cible s'hybridant spécifiquement à la séquence d'ADN cible ; et
(ii) une première étiquette d'affinité ;
la première séquence spécifique d'une cible de la première sonde et la deuxième séquence spécifique d'une cible de la deuxième sonde s'hybridant à des régions différentes du même ADN cible ;
(b) un deuxième nanorapporteur comprenant une troisième sonde et une quatrième sonde,
ladite troisième sonde comprenant
(i) une troisième région de rattachement de marqueurs à laquelle est/sont rattaché(s) un ou plusieurs monomère(s) marqueur(s) qui émet(tent) une lumière constituant un troisième signal ;
(ii) une quatrième région de rattachement de marqueurs qui ne chevauche pas la troisième région de rattachement de marqueurs, à laquelle est/sont rattaché(s) un ou plusieurs monomère(s) marqueur(s) qui émet(tent) une lumière constituant un quatrième signal ; et
(iii)une troisième séquence spécifique d'une cible rattachée à la troisième sonde, la troisième séquence spécifique d'une cible s'hybridant spécifiquement à un premier fragment d'ADN provenant d'une région du génome dont le nombre de copies ne varie pas ;
et ladite quatrième sonde comprenant
(i) une quatrième séquence spécifique d'une cible, la quatrième séquence spécifique d'une cible s'hybridant spécifiquement au premier fragment d'ADN provenant d'une région du génome dont le nombre de copies ne varie pas ; et
(ii) une deuxième étiquette d'affinité ;
la troisième séquence spécifique d'une cible de la troisième sonde et la quatrième séquence spécifique d'une cible de la quatrième sonde s'hybridant à des régions différentes du même fragment provenant d'une région du génome dont le nombre de copies ne varie pas ; et
(c) une enzyme de restriction.

14. Kit selon la revendication 13, dans lequel ledit premier fragment d'ADN provenant d'une région du génome dont le nombre de copies ne varie pas, auquel la troisième ou quatrième séquence cible s'hybride spécifiquement, comprend une molécule d'acide nucléique isolée comprenant au moins 50 nucléotides d'une séquence choisie dans l'ensemble constitué par les SEQ ID NO : 1 à 66.

15. Procédé selon l'une quelconque des revendications 1 à 4, comprenant en outre l'immobilisation et la purification de la première sonde hybridée au fragment devant être détecté par utilisation d'un réactif d'affinité qui se lie à l'étiquette d'affinité, en séparant ainsi la première sonde hybridée au fragment devant être détecté d'avec la sonde non liée ou l'ADN fragmenté.

16. Procédé selon l'une quelconque des revendications 1 à 12, dans lequel les ADN génomiques fragmentés de l'échantillon biologique et de l'échantillon de référence ont tous deux en moyenne entre 100 et 500 paires.
